# EUROPEAN PATENT APPLICATION

(11) **EP 1 136 553 A1**
(43) Date of publication of application: **26.09.2001**
(21) Application number: 00106225.6
(22) Date of filing: 22.03.2000
(51) Int. Cl.: C12N 15/12, C07K 14/755, C07K 14/745, C12N 15/57, C12N 9/64, A61K 38/36, A61K 38/37, A61K 38/43

(54) **Production of recombinant blood clotting factors in human cell lines**

(71) Applicant: Octagene GmbH, 80639 München (DE)
(72) Inventor: Hauser, Charlotte, 80369 München (DE); Hörster, Andrea, 80689 München (DE); Schröder, Carola, 81377 München (DE); Lehnerer, Michael, 80636 München (DE)
(74) Representative: Helbing, Jörg, Dr. Dipl.-Chem.

(57) **Abstract**

The present invention relates to a method for the production of recombinant human blood clotting factors, in particular of factor VIII and factor IX, utilizing an immortalized human cell line stably expressing a viral transcription activator protein and carrying a vector having a promoter functionally linked to a DNA sequence coding for a blood coagulating factor, wherein said promoter is not a viral promoter which is stimulated by said viral transcription activator protein; an immortalized human cell line carrying said vector; factor VIII muteins particularly suitable for the above production method; pharmaceutical compositions comprising such factor VIII muteins and the use of such factor VIII muteins for preparing a medicament for treating hemophilia.

## Description

### Introduction

The present invention relates to an improved method for the production of recombinant human blood clotting factors, in particular of factor VIII and factor IX, utilizing an immortalized human cell line stably expressing a viral transcription activator protein and carrying a vector having a promoter functionally linked to a DNA sequence coding for a blood coagulating factor, wherein said promoter is not a viral promoter which is stimulated by said viral transcription activator protein; an immortalized human cell line carrying said vector; factor VIII muteins particularly suitable for the above production method; pharmaceutical compositions comprising such factor VIII muteins and the use of such factor VIII muteins for preparing a medicament for treating hemophilia.

### Summary of the Related Art

Hemophiliacs are suffering from hemorrhagic morbidity caused by the disturbed function of protein components of the blood coagulation cascade. Dependent on the affected clotting factor two types of hemophilia can be distinguished. Both have in common the inhibited conversion of soluble fibrinogen to an insoluble fibrin-clot. They are recessive X-chromosomally-linked genetic diseases affecting mainly the male population.

Hemophilia A affects 1-2 individuals per 10.000 males. It is caused by the deficiency or absence of factor VIII, a very large glycoprotein (Mr approximately 360 kDa), which represents an important element of the blood coagulation cascade. The polypeptide sequence can be subdivided in three regions, an N-terminal region consisting of the so-called A1 and A2-domains, a central B-domain region and a C-terminal region composed of the A3, C1 and C2 domains. In the blood factor VIII occurs as an inactive precursor. It is bound tightly and non-covalently to von Willebrand Factor (vWF), which acts as a stabilizing carrier protein. Proteolytical cleavage of factor VIII by thrombin at three specific positions (740, 372, 1689) leads to its dissociation from vWF and releases the procoagulant function within the cascade. In its active form factor VIII functions as a cofactor for factor IXa, thereby accelerating the proteolytic activation of factor X by several orders of magnitude.
Hemophilia B occurs in about 1 of 25,000 males. It is characterized by the deficiency of the serine protease factor IX (Christmas factor). This 415 amino-acid polypeptide is synthesized in the liver as a 56kDa glycoprotein. In order to attain its proper function a posttranslational carboxylation step is required which only occurs in the presence of vitamin K.

Treatment of both types of bleeding disorder traditionally involves infusions of human plasma-derived protein concentrates of factor VIII or factor IX. Although this method represents an efficient therapy for hemophiliacs it carries the risk of transmission of various infectious agents, such as viruses causing hepatitis or HIV, or thromboembolic factors. Alternatively several recombinant DNA techniques for the production of clotting factors have been described. For this purpose the corresponding cDNAs of wild type factor VIII and factor IX have been isolated and cloned into suitable expression vectors (EP-A-160457; WO-A-86/01961, U.S. Patents 4,770,999, 5,521,070 and 5,521,070).

In the case of factor VIII recombinant expression of subunits for the production of complexes showing coagulant activity is known in the art (e.g., from EP-A-150735, EP-A-232112, EP-A-0500734, WO-91/07490, WO-95/13300 U.S. Patents 5,045,455 and 5,789,203). Moreover, the expression of truncated cDNA-versions partially or entirely lacking the sequence coding for the highly glycosylated B-domain have been described (e.g. in WO-86/06101, WO-87/04187, WO-87/07144, WO-88/00381, EP-A-251843, EP-A-253455, EP-A-254076, U.S. Patents 4,868,112 and 4,980,456, EP-A-294910, EP-A-265778, EP-A-303540 and WO-91/09122). More recently a variety of selected point mutations have been introduced to inhibit proteolytic inactivation of factor VIII by activated protein C or to reduce the immunogenicity resulting in the formation of inhibitory antibodies by the treated patients (e.g., U.S. Patents 5,859,204, 5,422,260 and 5,451,521, WO-97/49725 and WO-99/29848).

The recombinant clotting factors were usually isolated from the medium of stably transfected eucaryotic and preferably mammalian cell lines. In order to exclude the risk of copurifying some infectious agents which may be harbored by human cells non-human cell lines were employed in the production methods disclosed in the references mentioned herein before.

However, especially for factor VIII, the use of non-human cell lines encountered certain disadvantages. For example unsatisfactory secretion levels of the expressed protein into the medium has been reported. This may be due to slight differences within different types of mammalian cells concerning intracellular pathways for protein translation and modification, which also might have an effect on the biological activity of the expressed polypeptide. Apart from this, there were concerns that the therapeutic proteins purified from non-human expression systems are contaminated with cellular components which can give rise to antigenic reactions in the patients.

On the other hand, general methods for high level protein expression of a desired gene comprising immortalized, stably transfected mammalian cell lines expressing viral transcription activator proteins have been available for some time (e.g. U.S. Patent 5,712,119). Further these cell lines are transformed with a vector construct where a suitable viral transcription promoter is operatively associated with a DNA sequence defining a gene of interest, the transcription activator protein activates the viral transcription promoter and hence initiates the expression of the gene of interest. Again, there were concerns that the transcription activates expressed by these cell lines may give rise to contaminations in the target therapeutic protein.

In view of the above there was still a need for an effective production method for human blood clotting factors.

Surprisingly, it was found that the expression of a DNA encoding a blood clotting factor can be achieved with imortalized human cell lines wherein the cell lines carry a vector having a promoter functionally linked to said DNA sequence, despite the fact that the promoter is not a viral promoter stimulated by said viral transcription activator protein. In combination with suitable protein purification and virus-inactivation protocols this method provides an effective system to produce safe and highly active recombinant blood clotting factors for therapeutic applications in humans. Moreover, particular factor VIII muteins were found which are exceptionally stable against proteolytic inactivation and thus allow to be subjected to drastic virus inactivation protocols.

### Summary of the Invention

The present invention provides
(1) a method for the production of recombinant human blood clotting factor which comprises
   (a) culturing an immortalized human cell line stably expressing a viral transcription activator protein and carrying a vector having a promoter functionally linked to a DNA sequence coding for the human blood clotting factor, wherein said promoter is not a viral promoter which is stimulated by said viral transcription activator protein; and
   (b) isolating the blood clotting factor from the culture broth;
(2) a preferred embodiment of the method defined in (1) above, where the human blood clotting factor is factor IX or a mutein thereof;
(3) a preferred embodiment of the method defined in (1) above, where the human blood clotting factor is factor VIII or a mutein thereof;
(4) a preferred embodiment of the method defined in (3) above where the factor VIII is a mutein having at least one of the following mutations:
   Val at position 162 has been replaced by another neutral amino acid residue;
   Ser at position 2011 has been replaced by another hydrophilic amino acid residue; and
   Val at position 2223 has been replaced by an acidic amino acid residue, wherein said factor VIII numbering is relative to the amino acid sequence of wild-type factor VIII shown in SEQ ID NO: 3;
(5) an immortalized human cell line carrying a vector coding for a human blood clotting factor as defined in (1) to (4) above;
(6) a factor VIII mutein as defined in (4) above;
(7) a DNA coding for the factor VIII mutein as defined in (6) above;
(8) a vector comprising the DNA as defined in (6) above;
(9) a pharmaceutical composition comprising the factor VIII mutein as defined in (6) above;
(10) the use of the factor VIII mutein as defined in (6) above for preparing a medicament for treating hemophilia; and
(11) a method for treating hemophilia which comprises administering human hemophiliacs a factor VIII mutein as defined in (6) above.

### Description of the figures

Fig. 1 shows the fragments utilized for the construction of factor VIII with a deleted B-domain (Example 1).

Fig. 2 shows the vector pTGF8-1, 8720 bps circular DNA, the exact DNA sequence thereof is given in SEQ ID NO: 4 (for the factor VIII protein encoded by said DNA sequence see SEQ ID NO: 5).

Fig. 3 shows vector pTG36hyg, 8124 bps circular DNA.

Fig. 4 depicts a preferred linker sequence of the present invention (SEQ ID NO: 8).

Fig. 5 shows the coagulation time of recombinant hFVIII as determined in Example 7.

### Detailed Description of the Invention

"Functionally linked" refers to configurations of the vector where the promoter is located within the vector in such a manner that it can stimulate transcription of the DNA sequence coding for the human blood clotting factor. "Not functionally linked" refers to a configuration where the promoter is so remotely located from the blood clotting factor that it cannot stimulate its transcription.

"Gene" refers to a DNA sequence encoding a polypeptide optionally including leader and trailer sequences and introns and exons.

"Vector" refers to any genetic construct, such as plasmid, phage, cosmid, etc., which is capable of replication when associated with the proper control elements. The term includes cloning and expression vehicles.

"Promoter" refers to a region of regulatory DNA sequences for the control of transcription of a gene to which RNA polymerases bind.

"Therapeutically effective dose" of the pharmaceutical composition of the invention refers to a dose effective for treatment or prophylaxis, for example, a dose that yields effective treatment or reduction of the symptoms of hemophilia. The determination of a therapeutically effective dose is within the purview of one skilled in the art.

"Encodes" or "encoding" refers to a property of the nucleic acid sequence being transcribed (in case of DNA) or translated (in case mRNA) into a polypeptide *in vitro* or *in vivo* when placed under the control of an appropriate regulatory sequence.

For the purpose of this application "express", "expressing" or "expression" refers to the transcription and translation of a gene encoding a protein.

The present invention as described in (1) to (11) above is hereinafter described in more detail. In accordance with embodiment (1) of the invention of the present application the promoter functionally linked to the DNA sequence coding for the human blood clotting factor is not a viral promoter which is stimulated by the viral transcription activator protein expressed by the immortalized human cell line. The promoter functionally linked to the DNA sequence coding for the human blood clotting factor preferably is selected from viral promoters (such as SV40 and CMV), housekeeping host promoters (albumin), tissue specific promoters (such as α-antitrypsin for liver), etc., and most preferably is a CMV promoter. In accordance with the invention the vector may, however, carry viral promoters which are stimulated by said viral transcription activator protein, but which are not functionally linked to the blood clotting factor. Such viral promoters are selected from promoters derived from adenovirus, rous sarcoma virus and cyomegalovirus.

The immortalized human cell line embodiment (1) of the invention preferably is an immortalized kidney, bladder, liver, lung, cardiac muscle, smooth muscle, ovary or gastrointestinal cell. Preferably the immortalized human cell line is derived from embryonal human kidney cell, most preferably it is cell line 293 T (ECACC: tsa201, ref. 96121229).

In accordance with the present invention the vector may further comprise one or more of the following functional sequences: selection markers, regulatory sequences (e.g. PRE), etc.

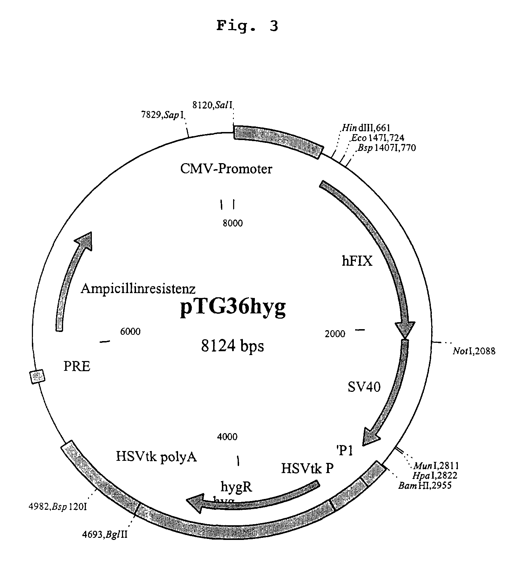

In preferred embodiment (2) of the present invention the human blood clotting factor is factor IX or a mutein thereof, preferably wild-type factor IX shown in SEQ ID NO: 1. Suitable muteins of factor IX include point mutated and truncated forms of the factor IX. The most preferred vector for expression of factor IX is vector pTG36hyg shown in Fig. 3.

In case of the production of factor IX, the culturing is preferably performed in the presence of vitamin K which may be present in an amount of 0.1 to 100 µg/ml culture broth, more preferably 1 to 20 µg/ml culture broth.

In preferred embodiment (3) of the invention the human blood clotting factor is factor VIII or a mutein thereof. Whereas recombinant factor IX is in general structurally identical to the wild type protein isolated from blood plasma, several modified factor VIII expression constructs have been designed for recombinant expression. Considering the domain structure of the functional factor VIII polypeptide important interaction sites with vWF are located in the A3-domain (amino acid 1680-1689) and in the C2-domain (Kaufman & Pipe, *Haemophilia* (1998) 4, 370-379). Cleavage after 1689 was proposed to liberate factor VIII from vWF and permit factor VIII to interact with charged phospholipids. Recombinant factor VIII constructs lacking the vWF-binding site were shown to be extremely prone to proteolytic digestion when injected into factor VIII-deficient mice. Recombinant expression of truncated factor VIII constructs in mammalian cell cultures demonstrated that the complete deletion of the B-domain did not alter the biological activity of the corresponding factor VIII-like protein (Eaton et. al., *Biochemistry* (1986) 25, 8343-8347). In addition the observed expression rates of B-domain deleted constructs were significantly higher compared to wild-type factor VIII due to an increased mRNA-level in the cells (Pittman et al., *Blood* (1993) 81, 2925-2935). Two recombinant factor VIII products (Recombinate® Baxter HealthCare; and Ko⁻ genate® Bayer Corporation) are currently on the market.

In preferred embodiment (4) of the invention the factor VIII mutein has at least one of the following mutations:
Val at position 162 has been replaced by another neutral amino acid residue;
Ser at position 2011 has been replaced by another hydrophilic amino acid residue; and
Val at position 2223 has been replaced by an acidic amino acid residue, wherein said factor VIII numbering is relative to the amino acid sequence of wild-type factor VIII shown in SEQ ID NO: 3 (being the amino acid sequence of the mature peptide not including the 19 amino acid signal peptide, but including the entire B-domain (WO-99/29848)).

"Another neutral amino acid residue" in accordance with the present invention includes Gly, Ala, Leu, Ile, Met and Pro and preferably is Ala. The "another hydrophilic amino acid" includes Asn, Thr and Gln and preferably is Asn. The acidic amino acid residue is selected from Glu and Asp and preferably is Glu.

It is preferred that the mutein comprises all three of the mutations V162A, S2011N and V2223E. In a most preferred embodiment the DNA sequence coding for factor VIII has the mutations T485C, G6032A and T6668A relative to the DNA sequence of wild-type factor VIII shown in SEQ ID NO: 2. In a preferred embodiment the DNA sequence also contains the quiet mutation T6816C (again said numbering being releative to the DNA sequence of wild-type factor VIII).

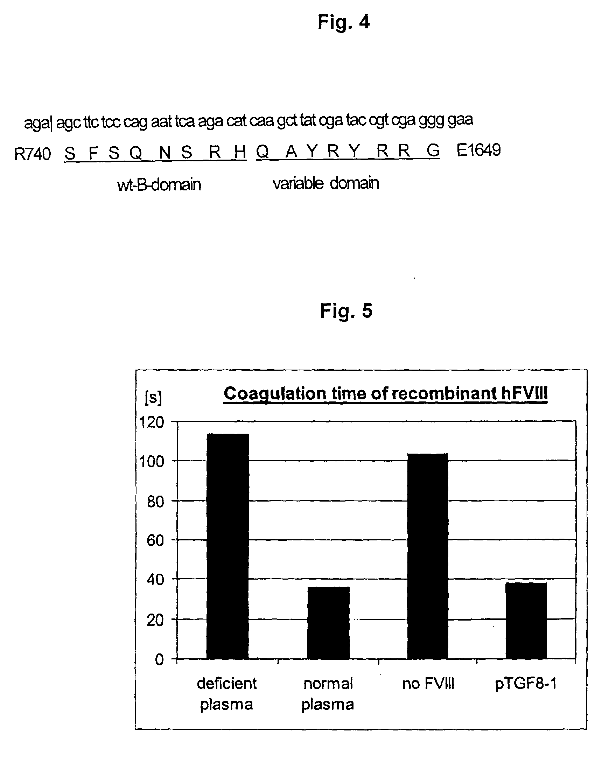

A preferred expression system of the invention utilizes a unique factor VIII in which the B-domain between position R740 and E1649 is replaced by a characteristic amino acid spacer. In accordance with the present invention said spacer possesses 10 to 25, preferably 14 to 20 amino acid residues. In a most preferred embodiment said spacer consists of eight amino acids of the wilde type B-domain followed by eight amino acids of a variable domain (see Fig. 4). In that construct the proposed vWF-binding site remains unchanged to prevent an immediate proteolytic digestion of secreted factor VIII in the cell culture medium or later on in the blood of the treated patients. Only after specific activation by thrombin cleavage factor VIII will be released from vWF. The cDNA for the preferred factor VIII was constructed by assembling four DNA-fragments obtained from a genbank.

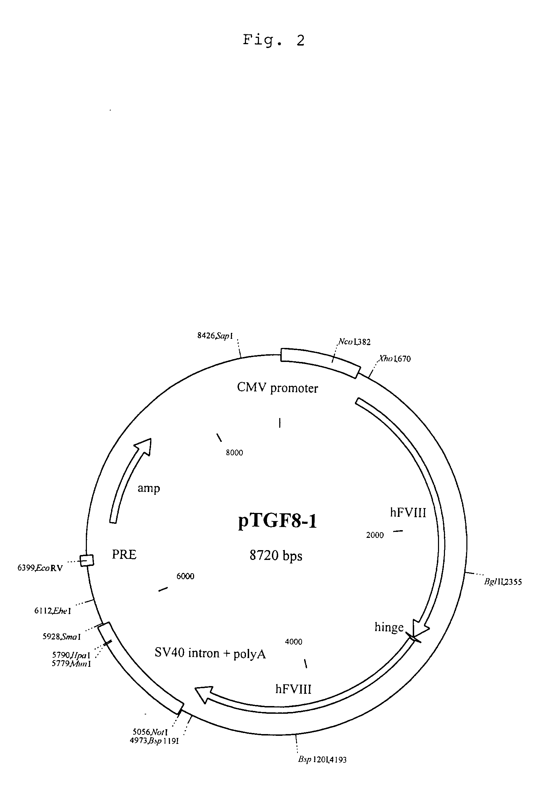

A most preferred vector for the expression of factor VIII is vector pTGF8-1 shown in Fig. 2. The DNA sequence of said vector is shown in SEQ ID NO: 4, and it encompasses all five mutations addressed hereinbefore (the muteins T485C, G6032A, T6668A and T6816C (here: T1217C, G4088A, T4724A and T4872C) and a DNA sequence coding for the B-domain linker of SEQ ID NO: 8) and encodes the factor VIII mutein depicted in SEQ ID NO:5.

In the case of the production of factor VIII the culturing is performed in the presence of von Willebrand factor. The von Willebrand factor is preferably used in an amount of 10 to 100, more preferably 50 to 60 mol vWF per mol factor VIII.

The method according to embodiment (1) of the invention further comprises the steps
(c) purifying the blood clotting factor isolated in step(b) and/or
(d) subjecting the blood clotting factor isolated in step (b) or purified in step (c) to a virus inactivation treatment.

Suitable purification steps are selected from immunoaffinity chromatography, anion exchange chromatography, size exclusion chromatography, etc., and combinations thereof. A virus inactivation treatment includes heat treatment (dry or in liquid state, with or without the addition of chemical substances including protease inhibitors). After virus inactivation a further purifying step for removing the chemical substances may be necessary.

The factor VIII mutein of embodiment (6) of the invention may further be prepared by a method comprising
(a) culturing an immortalized human cell line stably expressing a viral transcription activator protein and carrying a vector having a viral transcription promoter functionally linked to a DNA sequence coding for the human blood clotting factor, wherein said viral promoter is stimulated by said viral transcription activator protein; and
(b) isolating the blood clotting factor from the culture broth. Suitable viral promoters are those mentioned hereinbefore. The method may further comprise the purification and virus inactivation steps (c) and (d) described herein before.

The invention is further illustrated by the following examples.

### Examples

### Example 1 - Cloning of factor VIII and factor IX:

The sequence for the recombinant factor VIII was obtained by reverse transcription from a complete human hepatocellular RNA pool. Afterwords four fragments (1/2, 3/4 , 5/6, 7/8) were amplified by standard PCR using primers designed to contain restriction sites. To fit together the fragments 3/4 and 5/6 the SmaI/SalI fragment from plasmid pBSFVIII3/4 was inserted blunt into the SalI site of pBSFVII5/6 to obtain pBSFVIII3/6. Next the fragment 3/6 was obtained by digesting pBSFVIII3/6 with XhoI/BspHI and partially with

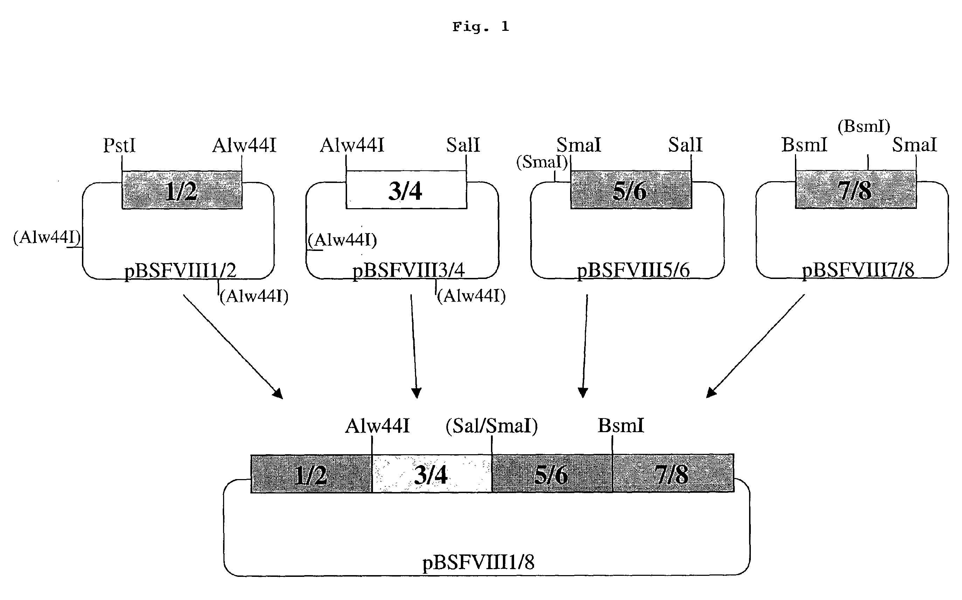

Alw44I. This fragment and the PstI/Alw44I fragment from pBSFVIII1/2 were ligated in one step into the vector backbone of pBSFVIII1/2 digested with PstI and XhoI by this means obtaining pBSFVIII1/6. The fragment 7/8 was obtained by digesting pBSFVIII7/8 with SmaI and partially with Mva1269I and ligated into pBSFVIII1/6 cut with XhoI and Mva1269I giving rise to pBSFVIII1/8. Finally the SmaI/XhoI fragment from pBSFIII1/8 was inserted blunt into the SalI site of Theragene Vector pTGFG67 (the production of said vector being disclosed in PCT/EP00/01368) resulting in the eucaryotic expression vector for the human factor VIII pTGF8-1 (s. figures 1 and 2). Surprisingly, the resulting vector, however, encoded a factor VIII mutein having the mutations V162A, S2011N an V2223E.

The isolation of human factor VIII cDNA and the production of a suitable expression vector for the reading frame corresponding to the human wild type factor IX is provided in PCT/EP00/01368 (e.g., the vector pTG36hyg shown in Fig. 3).

### Example 2 - Human cell line for protein expression:

A preferred cell line is tsA201 (ECACC Ref.: 96121229) which is an embryonal transformed human kidney cell line (293, ECACC Number 85120602) stably expressing an SV40 temperature-sensitive T antigen (J. Membrane Biol. 1996;152:39; Gene 1995;156:235; PNAS USA 1994;91:12785; Pflügers Arch. 1994;427:136; J. Gen. Physiol. 1994;104:507; BioTechniques 1993;15:906). Other names for this cell line include 293tsA1609neo (Mol. Cell. Biol., 1987, 7:379) and 293T. This epithelial-like cell line has been used in a variety of functional expression assays and been reported to produce high levels of recombinant proteins. They can be cultivated in DMEM supplemented with 2mM glutamine and 10% FCS. For efficient production fo factor IX the medium can be modified by addition of up to 100 µg/ml vitamin K (US4770999). To simplify the purification of an expressed polypetide cells can be cultivated in serum-free or protein-free medium containing suitable supplements. For stability reasons secreted factor VIII requires the presence of vWF in the medium (US5198349). Also the addition of lipoproteins, phospholipids, polyglycols, trace metals, heparin, non-ionic surfactants or cyclodextrin has been reported (EP0254076, US5679549, US5198349, US5250421, US5576194, EP0872487, WO94/11525, US5378612).

### Example 3 - Establishment of cell lines stably expressing factor VIII and factor IX:

The preferred vectors pTGF8-1 and pTG36hyg comprise constructs for transient expression of factor VIII and factor IX , respectively, in mammalian cells. To enable a selection method for a stably transfected cell clone, a cassette for the hygromycin―B-phosphotransferase (HindIII-Mva 1260I fragment from TK-Hyg, Clontech) was subcloned into the SmaI site beeing present in both vectors. The resulting constructs (pTGF8-1-hyg and pTG36hyg) then comprise in *cis* the expression cassettes for the human factor VIII or factor IX with a CMV-promotor and a SV40-polyadenylating signal and a hygromycin-B-phosphotransferase with the HSV thymidine kinase promoter and HSV thymidine kinase polyadenylating signal (s. figure 3). The coding sequence for the clotting factors can be replaced by any other gene sequence of choice. These constructs allow for the establishment of stably expressing cell lines by calcium phosphate transfection. Additionally the plasmids contain a progesterone responsive element. In transient transfection experiments with pTG36hyg the production of about 40 µg active factor IX per ml culture medium could be shown by coagulometric assay and ELISA (see Example 5).

293T cells were cultivated in DMEM supplemented with 10% FCS and 10 µg/ml Vitamin K (US4770999.). First the critical concentration of antibiotics for an effective selection of stably transfected 293T cells had to be established. For this purpose the cells were plated at low dilution and grown in the presence of 10 to 800 µg/ml hygromycin B. After two weeks at 200 µg/ml or higher no cells were growing, so this concentration was chosen for the selection of stably transfected cells.

A typical transfection was performed in 10cm dishes with 293T cells split the day before at a ratio of 1:15. By the calciumphosphate precipitation method (*Biotechniques* 1988 6:7 632-638) 12 µg plasmid per dish were transfected and two days later the medium was replaced with fresh one containing 200 g/ml hygromycin B. After 2-3 weeks of selection the medium was tested by ELISA (s. example 5) for the presence of factor IX. Positive clones were isolated and transferred to a 24-well plate. After screening by ELISA and coagulometer positive clones were subjected to two further rounds of subcloning then expanded and aliquots of them frozen for further use and characterization.

### Example 4 - Purification of coagulation factors:

The particular procedure for purification of the desired protein is selected from methods of the art to maximize the yield of a pure, stable and highly active product. Detailed purification protocols for coagulation factors from human blood plasma are available (WO93/15105, EP0813597, WO96/40883, WO 96/15140/50). They can easly be adapted to the specific requirements needed to isolate recombinant factors VIII and IX. Various means are available for extraction and purification of the expressed protein from the cell culture medium, such as immunoaffinity chromatography, ion exchange chromatography, size exclusion chromatography and the like. For factor IX an effective protocol has been introduced containing an ammonium sulfate precipitation step followed by DEAE and HIC tentacle chromatography as well as heparin affinity chromatography (US5919909). Quantity and activity of the purified protein during and after the purification procedure may be monitored by coagulation assays and ELISA.

### Example 5 - ELISA:

Human recombinant factor IX levels in supernatant of transfected 293T cells were determined by ELISA using a goat polyclonal anti-human FIX (Enzyme Research Laboratories) as capture antibody. All incubations were performed for two hours in a humid chamber at 22° C.

Plates (Dynex, Immulon-4) were coated with 100 µl of 8,8 µg antibody/ml coating buffer. Blocking is not required under the conditions described. Washing the plate four times (Encore 2000, Merck) with PBS-Tween® (0,1% w/w) is sufficient to block non-specific interactions. After each further step washing was required to eliminate unbound proteins. 100 µl of supernatant treated with 10 µl 10mM PMSF and 10 µl 0.11 M sodium citrate were added to each well. Dilutions for samples and standard (human factor IX, house standard, Octapharma) were made in dilution buffer (HBS-BSA-EDTA-Tween® ) and incubated at 100 µl/well. The detecting antibody was a peroxidase labelled goat polyclonal anti-FIX (Enzyme Research Laboratories) in a concentration of 1 µg antibody /ml dilution buffer and incubated at 100 µl/well. 150 µl ABTS (Roche) was added to each well as substrate, colorimetric reaction was detected at 405 nm after 1-2 hours. Results were calculated by linear regression of standard concentration versus standard absorbance and are summarized in the following Table:

| **Number of cells** **[/ml]** | **Factor IX-concentration** **[ng/ml]** | **Clotting time** **[s]** |
|---|---|---|
| 2,1 x 10⁵ | 36 | 45 |
| 8,7 x 10⁵ | 20 | 79 |

- Normal plasma:: 37 - 39 s
- Factor IX deficient plasma:: 137 - 140 s

The ELISA for factor VIII can be performed accordingly.

### Example 6 - Viral inactivation:

To overcome the problems of possible infectious contaminations in the purified protein samples the cell culture supernatant containing the secreted recombinant protein of choice might be subsequently treated with procedures for virus inactivation. For factor VIII isolated from blood plasma the recovery of a high purity virus-inactivated protein by anion exchange chromatography was described (WO93/15105).

In addition several processes for the production of high-purity, non-infectious coagulation factors from blood plasma or another biological sources have been reported. Lipid coated viruses are effectively inactivated by treating the potentially infectious material with a hydrophobic phase forming a two-phase system, from which the water-insoluble part is subsequently removed. A further advantage has been proven to complement the hydrophobic phase treatment simultaniously or sequentially with a treatment with non-ionic biocompatible detergents and dialkyl or trialkyl phosphates. (WO9636369, EP0131740). Non-lipid coated viruses require inactivation protocols consisting in treatment with non-ionic detergents follwed by a heating step (60-65 °C) for several hours (WO94/17834).

In view of the above results it is believed that the combination of an effective protein expression system based on a human cell line together with aproved methods for inactivation of potentially dangerous infectious agents serve as a safe and easy to use-system for production of a new generation of recombinant clotting factors.

### Example 7: Detection of Human Clotting Factor VIII an Factor IX activity from 293 T Cells Transfected with Calcium Phosphate

293T cells were transfected by calcium phosphate precipitation with pTGF8-1. This plasmid contains the cDNA of human clotting factor VIII. Recombinant human factor VIII was secreted into the supernatant of the cell culture and its activity determined.

Clotting activity was assayed based on a partial thromboplastin time assay using Cephalin (phosphatidyl ethanolamine) activation with a manual coagulation instrument (ML-2, Instrumentation Laboratories). For the study, 100 µl undiluted supernatant from transfected 293 T-cells, 100 µl deficiency plasma (Progen) and 100 µl Cephalin (Instrumentation Laboratories) were incubated for 5 minutes at 37°C. Coagulation was started by adding 100 µl CaCl₂. Sample coagulation time was compared to normal plasma. The results are summarized in Fig. 5. As can be seen from Fig. 5, cell supernatant from cells transfected with pTGF8-1 show a coagulation activity comparable to normal plasma while non transfected cells give a value equivalent to plasma lacking factor VIII.

A similar assay was performed with regard to factor IX. The results are shown in the table of Example 5.

## Claims

1. A method for the production of recombinant human blood clotting factor which comprises
(a) culturing an immortalized human cell line stably expressing a viral transcription activator protein and carrying a vector having a promoter functionally linked to a DNA sequence coding for the human blood clotting factor, wherein said promoter is not a viral promoter which is stimulated by said viral transcription activator protein; and
(b) isolating the blood clotting factor from the culture broth.

2. The method of claim 1, wherein the immortalized human cell line is an immortalized kidney, bladder, liver, lung, cardiac muscle, smooth muscle, ovary or gastrointestinal cell; and/or the promoter is selected from viral promoters, housekeeping host promoters and tissue specific promoters.

3. The method of claim 2, wherein the immortalized human cell line is derived from an embryonal human kidney cell and preferably is cell line 293T, and the promoter is a CMV promoter.

4. The method of claims 1 to 3, wherein the vector further comprises a selection marker and/or regulatory sequences.

5. The method of claims 1 to 4, wherein the human blood clotting factor is factor IX or a mutein thereof.

6. The method of claim 5, wherein the vector comprises a DNA sequence coding for the wild-type factor IX shown in SEQ ID NO: 1 and preferably is vector pTG36hyg shown in Fig. 3.

7. The method of claims 5 or 6, wherein the culturing is performed in the presence of vitamin K, preferably in an amount of 0.1 to 100 µg/ml, more preferably 1 to 20 µg/ml culture broth.

8. The method of claims 1 to 4, wherein the human blood clotting factor is factor VIII or a mutein thereof.

9. The method of claim 8, wherein the factor VIII mutein has at least one of the following mutations:
Val at position 162 has been replaced by another neutral amino acid residue;
Ser at position 2011 has been replaced by another hydrophilic amino acid residue; and
Val at position 2223 has been replaced by an acidic amino acid residue, wherein said factor VIII numbering is relative to the wild-type factor VIII shown in SEQ ID NO:2.

10. The method of claim 9, wherein the mutein comprises at least one of the mutations V162A, S2011N and V2223E, preferably all three of said mutations.

11. The method of claim 10, wherein the vector comprises a DNA sequence having at least one of the mutations T485C, G6032A and T6668A relative to the DNA sequence of wild-type factor VIII shown in SEQ ID NO: 2.

12. The method of claim 11, wherein the DNA sequence comprises all three of said mutations and preferably also contains the quiet mutation T6816C.

13. The method of claims 9 to 12, wherein the factor VIII mutein is a mutein partially or entirely lacking its B-domain.

14. The method of claim 13, wherein the factor VIII is a mutein where the B-domain between positions R740 and E1649 relative to the amino acid sequence of wild-type factor VIII shown in SEQ ID NO: 3 has been replaced by a linker peptide comprising 10 to 25, preferably 14 to 20 amino acid residues.

15. The method of claim 14, wherein the linker comprises:
the amino acid sequence SFSQNSRH, and/or
the amino acid sequence QAYRYRRG, and preferably the linker has
the sequence SFSQNSRHQAYRYRRG.

16. The method of claim 9, wherein the vector is pTGF8-1 shown in Fig. 2.

17. The method of claims 8 to 16, wherein the culturing is performed in the presence of von Willebrand Factor (vWF), preferably in an amount of 10 to 100, more preferably 50 to 60 mol vWF per mol factor VIII.

18. The method of claims 1 to 17 which further comprises
(c) purifying the blood clotting factor isolated in step (b), and/or
(d) subjecting the blood clotting factor isolated in step (b) or purified in step (c) to a virus inactivation treatment.

19. An immortalized human cell line stably expressing a viral transcription activator protein and carrying a vector coding for a human blood clotting factor as defined in claims 1 to 6 and 8 to 16.

20. A factor VIII mutein as defined in claims 9 to 16.

21. A DNA coding for the factor VIII mutein as defined in claim 20.

22. A vector comprising the DNA as defined in claim 21.

23. A pharmaceutical composition comprising the factor VIII mutein as defined in claim 20.

24. Use of the factor VIII mutein as defined in claim 20 for preparing a medicament for treating hemophilia.

25. A method for treating hemophilia which comprises administering human hemophiliacs a factor VIII mutein as defined in claim 20.


SEQUENCE LISTING

<110> Theragene Biomedical Laboratories GmbH

<120> Production of Recombinant Bood Clotting Factors in
      Human Cell Lines

<130> 992232ep/JH/ml

<140>
<141>

<160> 8

<170> PatentIn Ver. 2.1


<210> 1
<211> 461
<212> PRT
<213> Homo sapiens

<220>
<223> Description of Artificial Sequence: B-domain
      linker peptide

<400> 1


<210> 2
<211> 6996
<212> DNA
<213> Homo sapiens

<220>
<221> CDS
<222> (1)..(6996)

<400> 2
gcc acc aga aga tac tac ctg ggt gca gtg gaa ctg tca tgg gac tat      48
Ala Thr Arg Arg Tyr Tyr Leu Gly Ala Val Glu Leu Ser Trp Asp Tyr
1               5                   10                  15

atg caa agt gat ctc ggt gag ctg cct gtg gac gca aga ttt cct cct 96
Met Gln Ser Asp Leu Gly Glu Leu Pro Val Asp Ala Arg Phe Pro Pro
            20                  25                  30

aga gtg cca aaa tct ttt cca ttc aac acc tca gtc gtg tac aaa aag 144
Arg Val Pro Lys Ser Phe Pro Phe Asn Thr Ser Val Val Tyr Lys Lys
        35                  40                  45

act ctg ttt gta gaa ttc acg gtt cac ctt ttc aac atc gct aag cca 192
Thr Leu Phe Val Glu Phe Thr Val His Leu Phe Asn Ile Ala Lys Pro
    50                  55                  60

agg cca ccc tgg atg ggt ctg cta ggt cct acc atc cag gct gag gtt 240
Arg Pro Pro Trp Met Gly Leu Leu Gly Pro Thr Ile Gln Ala Glu Val
65                  70                  75                  80

tat gat aca gtg gtc att aca ctt aag aac atg gct tcc cat cct gtc 288
Tyr Asp Thr Val Val Ile Thr Leu Lys Asn Met Ala Ser His Pro Val
                85                  90                  95

agt ctt cat gct gtt ggt gta tcc tac tgg aaa gct tct gag gga gct 336
Ser Leu His Ala Val Gly Val Ser Tyr Trp Lys Ala Ser Glu Gly Ala
            100                 105                 110

gaa tat gat gat cag acc agt caa agg gag aaa gaa gat gat aaa gtc 384
Glu Tyr Asp Asp Gln Thr Ser Gln Arg Glu Lys Glu Asp Asp Lys Val
        115                 120                 125

ttc cct ggt gga agc cat aca tat gtc tgg cag gtc ctg aaa gag aat 432
Phe Pro Gly Gly Ser His Thr Tyr Val Trp Gln Val Leu Lys Glu Asn
    130                 135                 140

ggt cca atg gcc tct gac cca ctg tgc ctt acc tac tca tat ctt tct 480
Gly Pro Met Ala Ser Asp Pro Leu Cys Leu Thr Tyr Ser Tyr Leu Ser
145                 150                 155                 160

cat gtg gac ctg gta aaa gac ttg aat tca ggc ctc att gga gcc cta 528
His Val Asp Leu Val Lys Asp Leu Asn Ser Gly Leu Ile Gly Ala Leu
                165                 170                 175

cta gta tgt aga gaa ggg agt ctg gcc aag gaa aag aca cag acc ttg 576
Leu Val Cys Arg Glu Gly Ser Leu Ala Lys Glu Lys Thr Gln Thr Leu
            180                 185                 190

cac aaa ttt ata cta ctt ttt gct gta ttt gat gaa ggg aaa agt tgg 624
His Lys Phe Ile Leu Leu Phe Ala Val Phe Asp Glu Gly Lys Ser Trp
        195                 200                 205

cac tca gaa aca aag aac tcc ttg atg cag gat agg gat gct gca tct 672
His Ser Glu Thr Lys Asn Ser Leu Met Gln Asp Arg Asp Ala Ala Ser
    210                 215                 220

gct cgg gcc tgg cct aaa atg cac aca gtc aat ggt tat gta aac agg 720
Ala Arg Ala Trp Pro Lys Met His Thr Val Asn Gly Tyr Val Asn Arg
225                 230                 235                 240

tct ctg cca ggt ctg att gga tgc cac agg aaa tca gtc tat tgg cat 768
Ser Leu Pro Gly Leu Ile Gly Cys His Arg Lys Ser Val Tyr Trp His
                245                 250                 255

gtg att gga atg ggc acc act cct gaa gtg cac tca ata ttc ctc gaa 816
Val Ile Gly Met Gly Thr Thr Pro Glu Val His Ser Ile Phe Leu Glu
            260                 265                 270

ggt cac aca ttt ctt gtg agg aac cat cgc cag gcg tcc ttg gaa atc 864
Gly His Thr Phe Leu Val Arg Asn His Arg Gln Ala Ser Leu Glu Ile
        275                 280                 285

tcg cca ata act ttc ctt act gct caa aca ctc ttg atg gac ctt gga 912
Ser Pro Ile Thr Phe Leu Thr Ala Gln Thr Leu Leu Met Asp Leu Gly
    290                 295                 300

cag ttt cta ctg ttt tgt cat atc tct tcc cac caa cat gat ggc atg 960
Gln Phe Leu Leu Phe Cys His Ile Ser Ser His Gln His Asp Gly Met
305                 310                 315                 320

gaa gct tat gtc aaa gta gac agc tgt cca gag gaa ccc caa cta cga 1008
Glu Ala Tyr Val Lys Val Asp Ser Cys Pro Glu Glu Pro Gln Leu Arg
                325                 330                 335

atg aaa aat aat gaa gaa gcg gaa gac tat gat gat gat ctt act gat 1056
Met Lys Asn Asn Glu Glu Ala Glu Asp Tyr Asp Asp Asp Leu Thr Asp
            340                 345                 350

tct gaa atg gat gtg gtc agg ttt gat gat gac aac tct cct tcc ttt 1104
Ser Glu Met Asp Val Val Arg Phe Asp Asp Asp Asn Ser Pro Ser Phe
        355                 360                 365

atc caa att cgc tca gtt gcc aag aag cat cct aaa act tgg gta cat 1152
Ile Gln Ile Arg Ser Val Ala Lys Lys His Pro Lys Thr Trp Val His
    370                 375                 380

tac att gct gct gaa gag gag gac tgg gac tat gct ccc tta gtc ctc 1200
Tyr Ile Ala Ala Glu Glu Glu Asp Trp Asp Tyr Ala Pro Leu Val Leu
385                 390                 395                 400

gcc ccc gat gac aga agt tat aaa agt caa tat ttg aac aat ggc cct 1248
Ala Pro Asp Asp Arg Ser Tyr Lys Ser Gln Tyr Leu Asn Asn Gly Pro
                405                 410                 415

cag cgg att ggt agg aag tac aaa aaa gtc cga ttt atg gca tac aca 1296
Gln Arg Ile Gly Arg Lys Tyr Lys Lys Val Arg Phe Met Ala Tyr Thr
            420                 425                 430

gat gaa acc ttt aag act cgt gaa gct att cag cat gaa tca gga atc 1344
Asp Glu Thr Phe Lys Thr Arg Glu Ala Ile Gln His Glu Ser Gly Ile
        435                 440                 445

ttg gga cct tta ctt tat ggg gaa gtt gga gac aca ctg ttg att ata 1392
Leu Gly Pro Leu Leu Tyr Gly Glu Val Gly Asp Thr Leu Leu Ile Ile
    450                 455                 460

ttt aag aat caa gca agc aga cca tat aac atc tac cct cac gga atc 1440
Phe Lys Asn Gln Ala Ser Arg Pro Tyr Asn Ile Tyr Pro His Gly Ile
465                 470                 475                 480

act gat gtc cgt cct ttg tat tca agg aga tta cca aaa ggt gta aaa 1488
Thr Asp Val Arg Pro Leu Tyr Ser Arg Arg Leu Pro Lys Gly Val Lys
                485                 490                 495

cat ttg aag gat ttt cca att ctg cca gga gaa ata ttc aaa tat aaa 1536
His Leu Lys Asp Phe Pro Ile Leu Pro Gly Glu Ile Phe Lys Tyr Lys
            500                 505                 510

tgg aca gtg act gta gaa gat ggg cca act aaa tca gat cct cgg tgc 1584
Trp Thr Val Thr Val Glu Asp Gly Pro Thr Lys Ser Asp Pro Arg Cys
        515                 520                 525

ctg acc cgc tat tac tct agt ttc gtt aat atg gag aga gat cta gct 1632
Leu Thr Arg Tyr Tyr Ser Ser Phe Val Asn Met Glu Arg Asp Leu Ala
    530                 535                 540

tca gga ctc att ggc cct ctc ctc atc tgc tac aaa gaa tct gta gat 1680
Ser Gly Leu Ile Gly Pro Leu Leu Ile Cys Tyr Lys Glu Ser Val Asp
545                 550                 555                 560

caa aga gga aac cag ata atg tca gac aag agg aat gtc atc ctg ttt 1728
Gln Arg Gly Asn Gln Ile Met Ser Asp Lys Arg Asn Val Ile Leu Phe
                565                 570                 575

tct gta ttt gat gag aac cga agc tgg tac ctc aca gag aat ata caa 1776
Ser Val Phe Asp Glu Asn Arg Ser Trp Tyr Leu Thr Glu Asn Ile Gln
            580                 585                 590

cgc ttt ctc ccc aat cca gct gga gtg cag ctt gag gat cca gag ttc 1824
Arg Phe Leu Pro Asn Pro Ala Gly Val Gln Leu Glu Asp Pro Glu Phe
        595                 600                 605

caa gcc tcc aac atc atg cac agc atc aat ggc tat gtt ttt gat agt 1872
Gln Ala Ser Asn Ile Met His Ser Ile Asn Gly Tyr Val Phe Asp Ser
    610                 615                 620

ttg cag ttg tca gtt tgt ttg cat gag gtg gca tac tgg tac att cta 1920
Leu Gln Leu Ser Val Cys Leu His Glu Val Ala Tyr Trp Tyr Ile Leu
625                 630                 635                 640

agc att gga gca cag act gac ttc ctt tct gtc ttc ttc tct gga tat 1968
Ser Ile Gly Ala Gln Thr Asp Phe Leu Ser Val Phe Phe Ser Gly Tyr
                645                 650                 655

acc ttc aaa cac aaa atg gtc tat gaa gac aca ctc acc cta ttc cca 2016
Thr Phe Lys His Lys Met Val Tyr Glu Asp Thr Leu Thr Leu Phe Pro
            660                 665                 670

ttc tca gga gaa act gtc ttc atg tcg atg gaa aac cca ggt cta tgg 2064
Phe Ser Gly Glu Thr Val Phe Met Ser Met Glu Asn Pro Gly Leu Trp
        675                 680                 685

att ctg ggg tgc cac aac tca gac ttt cgg aac aga ggc atg acc gcc 2112
Ile Leu Gly Cys His Asn Ser Asp Phe Arg Asn Arg Gly Met Thr Ala
    690                 695                 700

tta ctg aag gtt tct agt tgt gac aag aac act ggt gat tat tac gag 2160
Leu Leu Lys Val Ser Ser Cys Asp Lys Asn Thr Gly Asp Tyr Tyr Glu
705                 710                 715                 720

gac agt tat gaa gat att tca gca tac ttg ctg agt aaa aac aat gcc 2208
Asp Ser Tyr Glu Asp Ile Ser Ala Tyr Leu Leu Ser Lys Asn Asn Ala
                725                 730                 735

att gaa cca aga agc ttc tcc cag aat tca aga cac cct agc act agg 2256
Ile Glu Pro Arg Ser Phe Ser Gln Asn Ser Arg His Pro Ser Thr Arg
            740                 745                 750

caa aag caa ttt aat gcc acc aca att cca gaa aat gac ata gag aag 2304
Gln Lys Gln Phe Asn Ala Thr Thr Ile Pro Glu Asn Asp Ile Glu Lys
        755                 760                 765

act gac cct tgg ttt gca cac aga aca cct atg cct aaa ata caa aat 2352
Thr Asp Pro Trp Phe Ala His Arg Thr Pro Met Pro Lys Ile Gln Asn
    770                 775                 780

gtc tcc tct agt gat ttg ttg atg ctc ttg cga cag agt cct act cca     2400
Val Ser Ser Ser Asp Leu Leu Met Leu Leu Arg Gln Ser Pro Thr Pro
785                 790                 795                 800

cat ggg cta tcc tta tct gat ctc caa gaa gcc aaa tat gag act ttt     2448
His Gly Leu Ser Leu Ser Asp Leu Gln Glu Ala Lys Tyr Glu Thr Phe
                805                 810                 815

tct gat gat cca tca cct gga gca ata gac agt aat aac agc ctg tct     2496
Ser Asp Asp Pro Ser Pro Gly Ala Ile Asp Ser Asn Asn Ser Leu Ser
            820                 825                 830

gaa atg aca cac ttc agg cca cag ctc cat cac agt ggg gac atg gta     2544
Glu Met Thr His Phe Arg Pro Gln Leu His His Ser Gly Asp Met Val
        835                 840                 845

ttt acc cct gag tca ggc ctc caa tta aga tta aat gag aaa ctg ggg     2592
Phe Thr Pro Glu Ser Gly Leu Gln Leu Arg Leu Asn Glu Lys Leu Gly
    850                 855                 860

aca act gca gca aca gag ttg aag aaa ctt gat ttc aaa gtt tct agt     2640
Thr Thr Ala Ala Thr Glu Leu Lys Lys Leu Asp Phe Lys Val Ser Ser
865                 870                 875                 880

aca tca aat aat ctg att tca aca att cca tca gac aat ttg gca gca     2688
Thr Ser Asn Asn Leu Ile Ser Thr Ile Pro Ser Asp Asn Leu Ala Ala
                885                 890                 895

ggt act gat aat aca agt tcc tta gga ccc cca agt atg cca gtt cat     2736
Gly Thr Asp Asn Thr Ser Ser Leu Gly Pro Pro Ser Met Pro Val His
            900                 905                 910

tat gat agt caa tta gat acc act cta ttt ggc aaa aag tca tct ccc     2784
Tyr Asp Ser Gln Leu Asp Thr Thr Leu Phe Gly Lys Lys Ser Ser Pro
        915                 920                 925

ctt act gag tct ggt gga cct ctg agc ttg agt gaa gaa aat aat gat     2832
Leu Thr Glu Ser Gly Gly Pro Leu Ser Leu Ser Glu Glu Asn Asn Asp
    930                 935                 940

tca aag ttg tta gaa tca ggt tta atg aat agc caa gaa agt tca tgg     2880
Ser Lys Leu Leu Glu Ser Gly Leu Met Asn Ser Gln Glu Ser Ser Trp
945                 950                 955                 960

gga aaa aat gta tcg tca aca gag agt ggt agg tta ttt aaa ggg aaa     2928
Gly Lys Asn Val Ser Ser Thr Glu Ser Gly Arg Leu Phe Lys Gly Lys
                965                 970                 975

aga gct cat gga cct gct ttg ttg act aaa gat aat gcc tta ttc aaa     2976
Arg Ala His Gly Pro Ala Leu Leu Thr Lys Asp Asn Ala Leu Phe Lys
            980                 985                 990

gtt agc atc tct ttg tta aag aca aac aaa act tcc aat aat tca gca     3024
Val Ser Ile Ser Leu Leu Lys Thr Asn Lys Thr Ser Asn Asn Ser Ala
        995                 1000                1005

act aat aga aag act cac att gat ggc cca tca tta tta att gag aat     3072
Thr Asn Arg Lys Thr His Ile Asp Gly Pro Ser Leu Leu Ile Glu Asn
    1010                1015                1020

agt cca tca gtc tgg caa aat ata tta gaa agt gac act gag ttt aaa     3120
Ser Pro Ser Val Trp Gln Asn Ile Leu Glu Ser Asp Thr Glu Phe Lys
1025                1030                1035                1040

aaa gtg aca cct ttg att cat gac aga atg ctt atg gac aaa aat gct    3168
Lys Val Thr Pro Leu Ile His Asp Arg Met Leu Met Asp Lys Asn Ala
            1045                1050                1055

aca gct ttg agg cta aat cat atg tca aat aaa act act tca tca aaa    3216
Thr Ala Leu Arg Leu Asn His Met Ser Asn Lys Thr Thr Ser Ser Lys
        1060                1065                1070

aac atg gaa atg gtc caa cag aaa aaa gag ggc ccc att cca cca gat    3264
Asn Met Glu Met Val Gln Gln Lys Lys Glu Gly Pro Ile Pro Pro Asp
    1075                1080                1085

gca caa aat cca gat atg tcg ttc ttt aag atg cta ttc ttg cca gaa    3312
Ala Gln Asn Pro Asp Met Ser Phe Phe Lys Met Leu Phe Leu Pro Glu
  1090                1095                1100

tca gca agg tgg ata caa agg act cat gga aag aac tct ctg aac tct    3360
Ser Ala Arg Trp Ile Gln Arg Thr His Gly Lys Asn Ser Leu Asn Ser
1105                1110                1115                1120

ggg caa ggc ccc agt cca aag caa tta gta tcc tta gga cca gaa aaa    3408
Gly Gln Gly Pro Ser Pro Lys Gln Leu Val Ser Leu Gly Pro Glu Lys
            1125                1130                1135

tct gtg gaa ggt cag aat ttc ttg tct gag aaa aac aaa gtg gta gta    3456
Ser Val Glu Gly Gln Asn Phe Leu Ser Glu Lys Asn Lys Val Val Val
        1140                1145                1150

gga aag ggt gaa ttt aca aag gac gta gga ctc aaa gag atg gtt ttt    3504
Gly Lys Gly Glu Phe Thr Lys Asp Val Gly Leu Lys Glu Met Val Phe
    1155                1160                1165

cca agc agc aga aac cta ttt ctt act aac ttg gat aat tta cat gaa    3552
Pro Ser Ser Arg Asn Leu Phe Leu Thr Asn Leu Asp Asn Leu His Glu
  1170                1175                1180

aat aat aca cac aat caa gaa aaa aaa att cag gaa gaa ata gaa aag    3600
Asn Asn Thr His Asn Gln Glu Lys Lys Ile Gln Glu Glu Ile Glu Lys
1185                1190                1195                1200

aag gaa aca tta atc caa gag aat gta gtt ttg cct cag ata cat aca    3648
Lys Glu Thr Leu Ile Gln Glu Asn Val Val Leu Pro Gln Ile His Thr
            1205                1210                1215

gtg act ggc act aag aat ttc atg aag aac ctt ttc tta ctg agc act    3696
Val Thr Gly Thr Lys Asn Phe Met Lys Asn Leu Phe Leu Leu Ser Thr
        1220                1225                1230

agg caa aat gta gaa ggt tca tat gag ggg gca tat gct cca gta ctt    3744
Arg Gln Asn Val Glu Gly Ser Tyr Glu Gly Ala Tyr Ala Pro Val Leu
    1235                1240                1245

caa gat ttt agg tca tta aat gat tca aca aat aga aca aag aaa cac    3792
Gln Asp Phe Arg Ser Leu Asn Asp Ser Thr Asn Arg Thr Lys Lys His
  1250                1255                1260

aca gct cat ttc tca aaa aaa ggg gag gaa gaa aac ttg gaa ggc ttg    3840
Thr Ala His Phe Ser Lys Lys Gly Glu Glu Glu Asn Leu Glu Gly Leu
1265                1270                1275                1280

gga aat caa acc aag caa att gta gag aaa tat gca tgc acc aca agg    3888
Gly Asn Gln Thr Lys Gln Ile Val Glu Lys Tyr Ala Cys Thr Thr Arg
            1285                1290                1295

ata tct cct aat aca agc cag cag aat ttt gtc acg caa cgt agt aag 3936
Ile Ser Pro Asn Thr Ser Gln Gln Asn Phe Val Thr Gln Arg Ser Lys
            1300                1305                1310

aga gct ttg aaa caa ttc aga ctc cca cta gaa gaa aca gaa ctt gaa 3984
Arg Ala Leu Lys Gln Phe Arg Leu Pro Leu Glu Glu Thr Glu Leu Glu
        1315                1320                1325

aaa agg ata att gtg gat gac acc tca acc cag tgg tcc aaa aac atg 4032
Lys Arg Ile Ile Val Asp Asp Thr Ser Thr Gln Trp Ser Lys Asn Met
    1330                1335                1340

aaa cat ttg acc ccg agc acc ctc aca cag ata gac tac aat gag aag 4080
Lys His Leu Thr Pro Ser Thr Leu Thr Gln Ile Asp Tyr Asn Glu Lys
1345                1350                1355                1360

gag aaa ggg gcc att act cag tct ccc tta tca gat tgc ctt acg agg 4128
Glu Lys Gly Ala Ile Thr Gln Ser Pro Leu Ser Asp Cys Leu Thr Arg
                1365                1370                1375

agt cat agc atc cct caa gca aat aga tct cca tta ccc att gca aag 4176
Ser His Ser Ile Pro Gln Ala Asn Arg Ser Pro Leu Pro Ile Ala Lys
            1380                1385                1390

gta tca tca ttt cca tct att aga cct ata tat ctg acc agg gtc cta 4224
Val Ser Ser Phe Pro Ser Ile Arg Pro Ile Tyr Leu Thr Arg Val Leu
        1395                1400                1405

ttc caa gac aac tct tct cat ctt cca gca gca tct tat aga aag aaa 4272
Phe Gln Asp Asn Ser Ser His Leu Pro Ala Ala Ser Tyr Arg Lys Lys
    1410                1415                1420

gat tct ggg gtc caa gaa agc agt cat ttc tta caa gga gcc aaa aaa 4320
Asp Ser Gly Val Gln Glu Ser Ser His Phe Leu Gln Gly Ala Lys Lys
1425                1430                1435                1440

aat aac ctt tct tta gcc att cta acc ttg gag atg act ggt gat caa 4368
Asn Asn Leu Ser Leu Ala Ile Leu Thr Leu Glu Met Thr Gly Asp Gln
                1445                1450                1455

aga gag gtt ggc tcc ctg ggg aca agt gcc aca aat tca gtc aca tac 4416
Arg Glu Val Gly Ser Leu Gly Thr Ser Ala Thr Asn Ser Val Thr Tyr
            1460                1465                1470

aag aaa gtt gag aac act gtt ctc ccg aaa cca gac ttg ccc aaa aca 4464
Lys Lys Val Glu Asn Thr Val Leu Pro Lys Pro Asp Leu Pro Lys Thr
        1475                1480                1485

tct ggc aaa gtt gaa ttg ctt cca aaa gtt cac att tat cag aag gac 4512
Ser Gly Lys Val Glu Leu Leu Pro Lys Val His Ile Tyr Gln Lys Asp
    1490                1495                1500

cta ttc cct acg gaa act agc aat ggg tct cct ggc cat ctg gat ctc 4560
Leu Phe Pro Thr Glu Thr Ser Asn Gly Ser Pro Gly His Leu Asp Leu
1505                1510                1515                1520

gtg gaa ggg agc ctt ctt cag gga aca gag gga gcg att aag tgg aat 4608
Val Glu Gly Ser Leu Leu Gln Gly Thr Glu Gly Ala Ile Lys Trp Asn
                1525                1530                1535

gaa gca aac aga cct gga aaa gtt ccc ttt ctg aga gta gca aca gaa 4656
Glu Ala Asn Arg Pro Gly Lys Val Pro Phe Leu Arg Val Ala Thr Glu
            1540                1545                1550

agc tct gca aag act ccc tcc aag cta ttg gat cct ctt gct tgg gat    4704
Ser Ser Ala Lys Thr Pro Ser Lys Leu Leu Asp Pro Leu Ala Trp Asp
        1555                1560                1565

aac cac tat ggt act cag ata cca aaa gaa gag tgg aaa tcc caa gag    4752
Asn His Tyr Gly Thr Gln Ile Pro Lys Glu Glu Trp Lys Ser Gln Glu
    1570                1575                1580

aag tca cca gaa aaa aca gct ttt aag aaa aag gat acc att ttg tcc    4800
Lys Ser Pro Glu Lys Thr Ala Phe Lys Lys Lys Asp Thr Ile Leu Ser
1585                1590                1595                1600

ctg aac gct tgt gaa agc aat cat gca ata gca gca ata aat gag gga    4848
Leu Asn Ala Cys Glu Ser Asn His Ala Ile Ala Ala Ile Asn Glu Gly
                1605                1610                1615

caa aat aag ccc gaa ata gaa gtc acc tgg gca aag caa ggt agg act    4896
Gln Asn Lys Pro Glu Ile Glu Val Thr Trp Ala Lys Gln Gly Arg Thr
            1620                1625                1630

gaa agg ctg tgc tct caa aac cca cca gtc ttg aaa cgc cat caa cgg    4944
Glu Arg Leu Cys Ser Gln Asn Pro Pro Val Leu Lys Arg His Gln Arg
        1635                1640                1645

gaa ata act cgt act act ctt cag tca gat caa gag gaa att gac tat    4992
Glu Ile Thr Arg Thr Thr Leu Gln Ser Asp Gln Glu Glu Ile Asp Tyr
    1650                1655                1660

gat gat acc ata tca gtt gaa atg aag aag gaa gat ttt gac att tat    5040
Asp Asp Thr Ile Ser Val Glu Met Lys Lys Glu Asp Phe Asp Ile Tyr
1665                1670                1675                1680

gat gag gat gaa aat cag agc ccc cgc agc ttt caa aag aaa aca cga    5088
Asp Glu Asp Glu Asn Gln Ser Pro Arg Ser Phe Gln Lys Lys Thr Arg
                1685                1690                1695

cac tat ttt att gct gca gtg gag agg ctc tgg gat tat ggg atg agt    5136
His Tyr Phe Ile Ala Ala Val Glu Arg Leu Trp Asp Tyr Gly Met Ser
            1700                1705                1710

agc tcc cca cat gtt cta aga aac agg gct cag agt ggc agt gtc cct    5184
Ser Ser Pro His Val Leu Arg Asn Arg Ala Gln Ser Gly Ser Val Pro
        1715                1720                1725

cag ttc aag aaa gtt gtt ttc cag gaa ttt act gat ggc tcc ttt act    5232
Gln Phe Lys Lys Val Val Phe Gln Glu Phe Thr Asp Gly Ser Phe Thr
    1730                1735                1740

cag ccc tta tac cgt gga gaa cta aat gaa cat ttg gga ctc ctg ggg    5280
Gln Pro Leu Tyr Arg Gly Glu Leu Asn Glu His Leu Gly Leu Leu Gly
1745                1750                1755                1760

cca tat ata aga gca gaa gtt gaa gat aat atc atg gta act ttc aga    5328
Pro Tyr Ile Arg Ala Glu Val Glu Asp Asn Ile Met Val Thr Phe Arg
                1765                1770                1775

aat cag gcc tct cgt ccc tat tcc ttc tat tct agc ctt att tct tat    5376
Asn Gln Ala Ser Arg Pro Tyr Ser Phe Tyr Ser Ser Leu Ile Ser Tyr
            1780                1785                1790

gag gaa gat cag agg caa gga gca gaa cct aga aaa aac ttt gtc aag    5424
Glu Glu Asp Gln Arg Gln Gly Ala Glu Pro Arg Lys Asn Phe Val Lys
        1795                1800                1805

cct aat gaa acc aaa act tac ttt tgg aaa gtg caa cat cat atg gca    5472
Pro Asn Glu Thr Lys Thr Tyr Phe Trp Lys Val Gln His His Met Ala
   1810                1815                1820

ccc act aaa gat gag ttt gac tgc aaa gcc tgg gct tat ttc tct gat    5520
Pro Thr Lys Asp Glu Phe Asp Cys Lys Ala Trp Ala Tyr Phe Ser Asp
1825                1830                1835                1840

gtt gac ctg gaa aaa gat gtg cac tca ggc ctg att gga ccc ctt ctg    5568
Val Asp Leu Glu Lys Asp Val His Ser Gly Leu Ile Gly Pro Leu Leu
                1845                1850                1855

gtc tgc cac act aac aca ctg aac cct gct cat ggg aga caa gtg aca    5616
Val Cys His Thr Asn Thr Leu Asn Pro Ala His Gly Arg Gln Val Thr
            1860                1865                1870

gta cag gaa ttt gct ctg ttt ttc acc atc ttt gat gag acc aaa agc    5664
Val Gln Glu Phe Ala Leu Phe Phe Thr Ile Phe Asp Glu Thr Lys Ser
        1875                1880                1885

tgg tac ttc act gaa aat atg gaa aga aac tgc agg gct ccc tgc aat    5712
Trp Tyr Phe Thr Glu Asn Met Glu Arg Asn Cys Arg Ala Pro Cys Asn
  . 1890                1895                1900

atc cag atg gaa gat ccc act ttt aaa gag aat tat cgc ttc cat gca    5760
Ile Gln Met Glu Asp Pro Thr Phe Lys Glu Asn Tyr Arg Phe His Ala
1905                1910                1915                1920

atc aat ggc tac ata atg gat aca cta cct ggc tta gta atg gct cag    5808
Ile Asn Gly Tyr Ile Met Asp Thr Leu Pro Gly Leu Val Met Ala Gln
                1925                1930                1935

gat caa agg att cga tgg tat ctg ctc agc atg ggc agc aat gaa aac    5856
Asp Gln Arg Ile Arg Trp Tyr Leu Leu Ser Met Gly Ser Asn Glu Asn
            1940                1945                1950

atc cat tct att cat ttc agt gga cat gtg ttc act gta cga aaa aaa    5904
Ile His Ser Ile His Phe Ser Gly His Val Phe Thr Val Arg Lys Lys
        1955                1960                1965

gag gag tat aaa atg gca ctg tac aat ctc tat cca ggt gtt ttt gag    5952
Glu Glu Tyr Lys Met Ala Leu Tyr Asn Leu Tyr Pro Gly Val Phe Glu
    1970                1975                1980

aca gtg gaa atg tta cca tcc aaa gct gga att tgg cgg gtg gaa tgc    6000
Thr Val Glu Met Leu Pro Ser Lys Ala Gly Ile Trp Arg Val Glu Cys
1985                1990                1995                2000

ctt att ggc gag cat cta cat gct ggg atg agc aca ctt ttt ctg gtg    6048
Leu Ile Gly Glu His Leu His Ala Gly Met Ser Thr Leu Phe Leu Val
                2005                2010                2015

tac agc aat aag tgt cag act ccc ctg gga atg gct tct gga cac att    6096
Tyr Ser Asn Lys Cys Gln Thr Pro Leu Gly Met Ala Ser Gly His Ile
            2020                2025                2030

aga gat ttt cag att aca gct tca gga caa tat gga cag tgg gcc cca    6144
Arg Asp Phe Gln Ile Thr Ala Ser Gly Gln Tyr Gly Gln Trp Ala Pro
        2035                2040                2045

aag ctg gcc aga ctt cat tat tcc gga tca atc aat gcc tgg agc acc    6192
Lys Leu Ala Arg Leu His Tyr Ser Gly Ser Ile Asn Ala Trp Ser Thr
    2050                2055                2060

aag gag ccc ttt tct tgg atc aag gtg gat ctg ttg gca cca atg att 6240
Lys Glu Pro Phe Ser Trp Ile Lys Val Asp Leu Leu Ala Pro Met Ile
2065 2070 2075 2080

att cac ggc atc aag acc cag ggt gcc cgt cag aag ttc tcc agc ctc 6288
Ile His Gly Ile Lys Thr Gln Gly Ala Arg Gln Lys Phe Ser Ser Leu
2085 2090 2095

tac atc tct cag ttt atc atc atg tat agt ctt gat ggg aag aag tgg 6336
Tyr Ile Ser Gln Phe Ile Ile Met Tyr Ser Leu Asp Gly Lys Lys Trp
2100 2105 2110

cag act tat cga gga aat tcc act gga acc tta atg gtc ttc ttt ggc 6384
Gln Thr Tyr Arg Gly Asn Ser Thr Gly Thr Leu Met Val Phe Phe Gly
2115 2120 2125

aat gtg gat tca tct ggg ata aaa cac aat att ttt aac cct cca att 6432
Asn Val Asp Ser Ser Gly Ile Lys His Asn Ile Phe Asn Pro Pro Ile
2130 2135 2140

att gct cga tac atc cgt ttg cac cca act cat tat agc att cgc agc 6480
Ile Ala Arg Tyr Ile Arg Leu His Pro Thr His Tyr Ser Ile Arg Ser
2145 2150 2155 2160

act ctt cgc atg gag ttg atg ggc tgt gat tta aat agt tgc agc atg 6528
Thr Leu Arg Met Glu Leu Met Gly Cys Asp Leu Asn Ser Cys Ser Met
2165 2170 2175

cca ttg gga atg gag agt aaa gca ata tca gat gca cag att act gct 6576
Pro Leu Gly Met Glu Ser Lys Ala Ile Ser Asp Ala Gln Ile Thr Ala
2180 2185 2190

tca tcc tac ttt acc aat atg ttt gcc acc tgg tct cct tca aaa gct 6624
Ser Ser Tyr Phe Thr Asn Met Phe Ala Thr Trp Ser Pro Ser Lys Ala
2195 2200 2205

cga ctt cac ctc caa ggg agg agt aat gcc tgg aga cct cag gtg aat 6672
Arg Leu His Leu Gln Gly Arg Ser Asn Ala Trp Arg Pro Gln Val Asn
2210 2215 2220

aat cca aaa gag tgg ctg caa gtg gac ttc cag aag aca atg aaa gtc 6720
Asn Pro Lys Glu Trp Leu Gln Val Asp Phe Gln Lys Thr Met Lys Val
2225 2230 2235 2240

aca gga gta act act cag gga gta aaa tct ctg ctt acc agc atg tat 6768
Thr Gly Val Thr Thr Gln Gly Val Lys Ser Leu Leu Thr Ser Met Tyr
2245 2250 2255

gtg aag gag ttc ctc atc tcc agc agt caa gat ggc cat cag tgg act 6816
Val Lys Glu Phe Leu Ile Ser Ser Ser Gln Asp Gly His Gln Trp Thr
2260 2265 2270

ctc ttt ttt cag aat ggc aaa gta aag gtt ttt cag gga aat caa gac 6864
Leu Phe Phe Gln Asn Gly Lys Val Lys Val Phe Gln Gly Asn Gln Asp
2275 2280 2285

tcc ttc aca cct gtg gtg aac tct cta gac cca ccg tta ctg act cgc 6912
Ser Phe Thr Pro Val Val Asn Ser Leu Asp Pro Pro Leu Leu Thr Arg
2290 2295 2300

tac ctt cga att cac ccc cag agt tgg gtg cac cag att gcc ctg agg 6960
Tyr Leu Arg Ile His Pro Gln Ser Trp Val His Gln Ile Ala Leu Arg
2305 2310 2315 2320

atg gag gtt ctg ggc tgc gag gca cag gac ctc tac 6996
Met Glu Val Leu Gly Cys Glu Ala Gln Asp Leu Tyr
2325 2330

<210> 3
<211> 2332
<212> PRT
<213> Homo sapiens

<400> 3
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Val His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His
            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Ser Thr Glu Ser Gly Arg Leu Phe Lys Gly Lys
                965                 970                 975

Arg Ala His Gly Pro Ala Leu Leu Thr Lys Asp Asn Ala Leu Phe Lys
            980                 985                 990

Val Ser Ile Ser Leu Leu Lys Thr Asn Lys Thr Ser Asn Asn Ser Ala
        995                 1000                1005

Thr Asn Arg Lys Thr His Ile Asp Gly Pro Ser Leu Leu Ile Glu Asn
   1010                1015                1020

Ser Pro Ser Val Trp Gln Asn Ile Leu Glu Ser Asp Thr Glu Phe Lys
025                 1030                1035                1040

Lys Val Thr Pro Leu Ile His Asp Arg Met Leu Met Asp Lys Asn Ala
                1045                1050                1055

Thr Ala Leu Arg Leu Asn His Met Ser Asn Lys Thr Thr Ser Ser Lys
            1060                1065                1070

Asn Met Glu Met Val Gln Gln Lys Lys Glu Gly Pro Ile Pro Pro Asp
        1075                1080                1085

Ala Gln Asn Pro Asp Met Ser Phe Phe Lys Met Leu Phe Leu Pro Glu
   1090                1095                1100

Ser Ala Arg Trp Ile Gln Arg Thr His Gly Lys Asn Ser Leu Asn Ser
105                 1110                1115                1120

Gly Gln Gly Pro Ser Pro Lys Gln Leu Val Ser Leu Gly Pro Glu Lys
                1125                1130                1135

Ser Val Glu Gly Gln Asn Phe Leu Ser Glu Lys Asn Lys Val Val Val
            1140                1145                1150

Gly Lys Gly Glu Phe Thr Lys Asp Val Gly Leu Lys Glu Met Val Phe
        1155                1160                1165

Pro Ser Ser Arg Asn Leu Phe Leu Thr Asn Leu Asp Asn Leu His Glu
   1170                1175                1180

Asn Asn Thr His Asn Gln Glu Lys Lys Ile Gln Glu Glu Ile Glu Lys
185                 1190                1195                1200

Lys Glu Thr Leu Ile Gln Glu Asn Val Val Leu Pro Gln Ile His Thr
                1205                1210                1215

Val Thr Gly Thr Lys Asn Phe Met Lys Asn Leu Phe Leu Leu Ser Thr
            1220                1225                1230

Arg Gln Asn Val Glu Gly Ser Tyr Glu Gly Ala Tyr Ala Pro Val Leu
        1235                1240                1245

Gln Asp Phe Arg Ser Leu Asn Asp Ser Thr Asn Arg Thr Lys Lys His
   1250                1255                1260

Thr Ala His Phe Ser Lys Lys Gly Glu Glu Glu Asn Leu Glu Gly Leu
265                 1270                1275                1280

Gly Asn Gln Thr Lys Gln Ile Val Glu Lys Tyr Ala Cys Thr Thr Arg
                1285                1290                1295

Ile Ser Pro Asn Thr Ser Gln Gln Asn Phe Val Thr Gln Arg Ser Lys
            1300                1305                1310

Arg Ala Leu Lys Gln Phe Arg Leu Pro Leu Glu Glu Thr Glu Leu Glu
        1315                1320                1325

Lys Arg Ile Ile Val Asp Asp Thr Ser Thr Gln Trp Ser Lys Asn Met
   1330                1335                1340

Lys His Leu Thr Pro Ser Thr Leu Thr Gln Ile Asp Tyr Asn Glu Lys
345                 1350                1355                1360

Glu Lys Gly Ala Ile Thr Gln Ser Pro Leu Ser Asp Cys Leu Thr Arg
                1365                1370                1375

Ser His Ser Ile Pro Gln Ala Asn Arg Ser Pro Leu Pro Ile Ala Lys
            1380                1385                1390

Val Ser Ser Phe Pro Ser Ile Arg Pro Ile Tyr Leu Thr Arg Val Leu
        1395                1400                1405

Phe Gln Asp Asn Ser Ser His Leu Pro Ala Ala Ser Tyr Arg Lys Lys
    1410                1415                1420

Asp Ser Gly Val Gln Glu Ser Ser His Phe Leu Gln Gly Ala Lys Lys
425                 1430                1435                1440

Asn Asn Leu Ser Leu Ala Ile Leu Thr Leu Glu Met Thr Gly Asp Gln
                1445                1450                1455

Arg Glu Val Gly Ser Leu Gly Thr Ser Ala Thr Asn Ser Val Thr Tyr
            1460                1465                1470

Lys Lys Val Glu Asn Thr Val Leu Pro Lys Pro Asp Leu Pro Lys Thr
        1475                1480                1485

Ser Gly Lys Val Glu Leu Leu Pro Lys Val His Ile Tyr Gln Lys Asp
    1490                1495                1500

Leu Phe Pro Thr Glu Thr Ser Asn Gly Ser Pro Gly His Leu Asp Leu
505                 1510                1515                1520

Val Glu Gly Ser Leu Leu Gln Gly Thr Glu Gly Ala Ile Lys Trp Asn
                1525                1530                1535

Glu Ala Asn Arg Pro Gly Lys Val Pro Phe Leu Arg Val Ala Thr Glu
            1540                1545                1550

Ser Ser Ala Lys Thr Pro Ser Lys Leu Leu Asp Pro Leu Ala Trp Asp
        1555                1560                1565

Asn His Tyr Gly Thr Gln Ile Pro Lys Glu Glu Trp Lys Ser Gln Glu
    1570                1575                1580

Lys Ser Pro Glu Lys Thr Ala Phe Lys Lys Lys Asp Thr Ile Leu Ser
585                 1590                1595                1600

Leu Asn Ala Cys Glu Ser Asn His Ala Ile Ala Ala Ile Asn Glu Gly
                1605                1610                1615

Gln Asn Lys Pro Glu Ile Glu Val Thr Trp Ala Lys Gln Gly Arg Thr
            1620                1625                1630

Glu Arg Leu Cys Ser Gln Asn Pro Pro Val Leu Lys Arg His Gln Arg
        1635                1640                1645

Glu Ile Thr Arg Thr Thr Leu Gln Ser Asp Gln Glu Glu Ile Asp Tyr
    1650                1655                1660

Asp Asp Thr Ile Ser Val Glu Met Lys Lys Glu Asp Phe Asp Ile Tyr
665                 1670                1675                1680

Asp Glu Asp Glu Asn Gln Ser Pro Arg Ser Phe Gln Lys Lys Thr Arg
                1685                1690                1695

His Tyr Phe Ile Ala Ala Val Glu Arg Leu Trp Asp Tyr Gly Met Ser
            1700                1705                1710

Ser Ser Pro His Val Leu Arg Asn Arg Ala Gln Ser Gly Ser Val Pro
        1715                1720                1725

Gln Phe Lys Lys Val Val Phe Gln Glu Phe Thr Asp Gly Ser Phe Thr
    1730                1735                1740

Gln Pro Leu Tyr Arg Gly Glu Leu Asn Glu His Leu Gly Leu Leu Gly
745                 1750                1755                1760

Pro Tyr Ile Arg Ala Glu Val Glu Asp Asn Ile Met Val Thr Phe Arg
                1765                1770                1775

Asn Gln Ala Ser Arg Pro Tyr Ser Phe Tyr Ser Ser Leu Ile Ser Tyr
            1780                1785                1790

Glu Glu Asp Gln Arg Gln Gly Ala Glu Pro Arg Lys Asn Phe Val Lys
        1795                1800                1805

Pro Asn Glu Thr Lys Thr Tyr Phe Trp Lys Val Gln His His Met Ala
    1810                1815                1820

Pro Thr Lys Asp Glu Phe Asp Cys Lys Ala Trp Ala Tyr Phe Ser Asp
825                 1830                1835                1840

Val Asp Leu Glu Lys Asp Val His Ser Gly Leu Ile Gly Pro Leu Leu
                1845                1850                1855

Val Cys His Thr Asn Thr Leu Asn Pro Ala His Gly Arg Gln Val Thr
            1860                1865                1870

Val Gln Glu Phe Ala Leu Phe Phe Thr Ile Phe Asp Glu Thr Lys Ser
        1875                1880                1885

Trp Tyr Phe Thr Glu Asn Met Glu Arg Asn Cys Arg Ala Pro Cys Asn
    1890                1895                1900

Ile Gln Met Glu Asp Pro Thr Phe Lys Glu Asn Tyr Arg Phe His Ala
905                 1910                1915                1920

Ile Asn Gly Tyr Ile Met Asp Thr Leu Pro Gly Leu Val Met Ala Gln
                1925                1930                1935

Asp Gln Arg Ile Arg Trp Tyr Leu Leu Ser Met Gly Ser Asn Glu Asn
            1940                1945                1950

Ile His Ser Ile His Phe Ser Gly His Val Phe Thr Val Arg Lys Lys
        1955                1960                1965

Glu Glu Tyr Lys Met Ala Leu Tyr Asn Leu Tyr Pro Gly Val Phe Glu
    1970                1975                1980

Thr Val Glu Met Leu Pro Ser Lys Ala Gly Ile Trp Arg Val Glu Cys
985                 1990                1995                2000

Leu Ile Gly Glu His Leu His Ala Gly Met Ser Thr Leu Phe Leu Val
                2005                2010                2015

Tyr Ser Asn Lys Cys Gln Thr Pro Leu Gly Met Ala Ser Gly His Ile
            2020                2025                2030

Arg Asp Phe Gln Ile Thr Ala Ser Gly Gln Tyr Gly Gln Trp Ala Pro
        2035                2040                2045

Lys Leu Ala Arg Leu His Tyr Ser Gly Ser Ile Asn Ala Trp Ser Thr
    2050                2055                2060

Lys Glu Pro Phe Ser Trp Ile Lys Val Asp Leu Leu Ala Pro Met Ile
065                 2070                2075                2080

Ile His Gly Ile Lys Thr Gln Gly Ala Arg Gln Lys Phe Ser Ser Leu
                2085                2090                2095

Tyr Ile Ser Gln Phe Ile Ile Met Tyr Ser Leu Asp Gly Lys Lys Trp
            2100                2105                2110

Gln Thr Tyr Arg Gly Asn Ser Thr Gly Thr Leu Met Val Phe Phe Gly
        2115                2120                2125

Asn Val Asp Ser Ser Gly Ile Lys His Asn Ile Phe Asn Pro Pro Ile
    2130                2135                2140

Ile Ala Arg Tyr Ile Arg Leu His Pro Thr His Tyr Ser Ile Arg Ser
145                 2150                2155                2160

Thr Leu Arg Met Glu Leu Met Gly Cys Asp Leu Asn Ser Cys Ser Met
                2165                2170                2175

Pro Leu Gly Met Glu Ser Lys Ala Ile Ser Asp Ala Gln Ile Thr Ala
            2180                2185                2190

Ser Ser Tyr Phe Thr Asn Met Phe Ala Thr Trp Ser Pro Ser Lys Ala
        2195                2200                2205

Arg Leu His Leu Gln Gly Arg Ser Asn Ala Trp Arg Pro Gln Val Asn
    2210                2215                2220

Asn Pro Lys Glu Trp Leu Gln Val Asp Phe Gln Lys Thr Met Lys Val
225                 2230                2235                2240

Thr Gly Val Thr Thr Gln Gly Val Lys Ser Leu Leu Thr Ser Met Tyr
                2245                2250                2255

Val Lys Glu Phe Leu Ile Ser Ser Ser Gln Asp Gly His Gln Trp Thr
            2260                2265                2270

Leu Phe Phe Gln Asn Gly Lys Val Lys Val Phe Gln Gly Asn Gln Asp
        2275                2280                2285

Ser Phe Thr Pro Val Val Asn Ser Leu Asp Pro Pro Leu Leu Thr Arg
    2290                2295                2300

Tyr Leu Arg Ile His Pro Gln Ser Trp Val His Gln Ile Ala Leu Arg
305                 2310                2315                2320

Met Glu Val Leu Gly Cys Glu Ala Gln Asp Leu Tyr
                2325                2330


<210> 4
<211> 8720
<212> DNA
<213> Artificial Sequence

<220>
<223> Description of Artificial Sequence: pTGF8-1

<220>
<221> CDS
<222> (676)..(5052)

<400> 4
cgcgttgaca ttgattattg actagttatt aatagtaatc aattacgggg tcattagttc 60

atagcccata tatggagttc cgcgttacat aacttacggt aaatggcccg cctggctgac 120

cgcccaacga cccccgccca ttgacgtcaa taatgacgta tgttcccata gtaacgccaa 180

tagggacttt ccattgacgt caatgggtgg agtatttacg gtaaactgcc cacttggcag 240

tacatcaagt gtatcatatg ccaagtacgc cccctattga cgtcaatgac ggtaaatggc 300

ccgcctggca ttatgcccag tacatgacct tatgggactt tcctacttgg cagtacatct 360

acgtattagt catcgctatt accatggtga tgcggttttg gcagtacatc aatgggcgtg 420

gatagcggtt tgactcacgg ggatttccaa gtctccaccc cattgacgtc aatgggagtt 480

tgttttggca ccaaaatcaa cgggactttc caaaatgtcg taacaactcc gccccattga 540

cgcaaatggg cggtaggcgt gtacggtggg aggtctatat aagcagagct ctctggctaa 600

ctagagaacc cactgcttac tggcttatcg aaattaatac gactcactat agggagaccc 660

aagcttgacc tcgag atg caa ata gag ctc tcc acc tgc ttc ttt ctg tgc 711
Met Gln Ile Glu Leu Ser Thr Cys Phe Phe Leu Cys
1               5                   10

ctt ttg cga ttc tgc ttt agt gcc acc aga aga tac tac ctg ggt gca 759
Leu Leu Arg Phe Cys Phe Ser Ala Thr Arg Arg Tyr Tyr Leu Gly Ala
        15                  20                  25

gtg gaa ctg tca tgg gac tat atg caa agt gat ctc ggt gag ctg cct 807
Val Glu Leu Ser Trp Asp Tyr Met Gln Ser Asp Leu Gly Glu Leu Pro
    30                  35                  40

gtg gac gca aga ttt cct cct aga gtg cca aaa tct ttt cca ttc aac 855
Val Asp Ala Arg Phe Pro Pro Arg Val Pro Lys Ser Phe Pro Phe Asn
45                  50                  55                  60

acc tca gtc gtg tac aaa aag act ctg ttt gta gaa ttc acg gat cac 903
Thr Ser Val Val Tyr Lys Lys Thr Leu Phe Val Glu Phe Thr Asp His
                65                  70                  75

ctt ttc aac atc gct aag cca agg cca ccc tgg atg ggt ctg cta ggt 951
Leu Phe Asn Ile Ala Lys Pro Arg Pro Pro Trp Met Gly Leu Leu Gly
            80                  85                  90

cct acc atc cag gct gag gtt tat gat aca gtg gtc att aca ctt aag 999
Pro Thr Ile Gln Ala Glu Val Tyr Asp Thr Val Val Ile Thr Leu Lys
        95                  100                 105

aac atg gct tcc cat cct gtc agt ctt cat gct gtt ggt gta tcc tac 1047
Asn Met Ala Ser His Pro Val Ser Leu His Ala Val Gly Val Ser Tyr
    110                 115                 120

tgg aaa gct tct gag gga gct gaa tat gat gat cag acc agt caa agg 1095
Trp Lys Ala Ser Glu Gly Ala Glu Tyr Asp Asp Gln Thr Ser Gln Arg
125                 130                 135                 140

gag aaa gaa gat gat aaa gtc ttc cct ggt gga agc cat aca tat gtc 1143
Glu Lys Glu Asp Asp Lys Val Phe Pro Gly Gly Ser His Thr Tyr Val
                145                 150                 155

tgg cag gtc ctg aaa gag aat ggt cca atg gcc tct gac cca ctg tgc    1191
Trp Gln Val Leu Lys Glu Asn Gly Pro Met Ala Ser Asp Pro Leu Cys
            160                 165                 170

ctt acc tac tca tat ctt tct cat gcg gac ctg gta aaa gac ttg aat    1239
Leu Thr Tyr Ser Tyr Leu Ser His Ala Asp Leu Val Lys Asp Leu Asn
        175                 180                 185

tca ggc ctc att gga gcc cta cta gta tgt aga gaa ggg agt ctg gcc    1287
Ser Gly Leu Ile Gly Ala Leu Leu Val Cys Arg Glu Gly Ser Leu Ala
    190                 195                 200

aag gaa aag aca cag acc ttg cac aaa ttt ata cta ctt ttt gct gta    1335
Lys Glu Lys Thr Gln Thr Leu His Lys Phe Ile Leu Leu Phe Ala Val
205                 210                 215                 220

ttt gat gaa ggg aaa agt tgg cac tca gaa aca aag aac tcc ttg atg    1383
Phe Asp Glu Gly Lys Ser Trp His Ser Glu Thr Lys Asn Ser Leu Met
                225                 230                 235

cag gat agg gat gct gca tct gct cgg gcc tgg cct aaa atg cac aca    1431
Gln Asp Arg Asp Ala Ala Ser Ala Arg Ala Trp Pro Lys Met His Thr
            240                 245                 250

gtc aat ggt tat gta aac agg tct ctg cca ggt ctg att gga tgc cac    1479
Val Asn Gly Tyr Val Asn Arg Ser Leu Pro Gly Leu Ile Gly Cys His
        255                 260                 265

agg aaa tca gtc tat tgg cat gtg att gga atg ggc acc act cct gaa    1527
Arg Lys Ser Val Tyr Trp His Val Ile Gly Met Gly Thr Thr Pro Glu
    270                 275                 280

gtg cac tca ata ttc ctc gaa ggt cac aca ttt ctt gtg agg aac cat    1575
Val His Ser Ile Phe Leu Glu Gly His Thr Phe Leu Val Arg Asn His
285                 290                 295                 300

cgc cag gcg tcc ttg gaa atc tcg cca ata act ttc ctt act gct caa    1623
Arg Gln Ala Ser Leu Glu Ile Ser Pro Ile Thr Phe Leu Thr Ala Gln
                305                 310                 315

aca ctc ttg atg gac ctt gga cag ttt cta ctg ttt tgt cat atc tct    1671
Thr Leu Leu Met Asp Leu Gly Gln Phe Leu Leu Phe Cys His Ile Ser
            320                 325                 330

tcc cac caa cat gat ggc atg gaa gct tat gtc aaa gta gac agc tgt    1719
Ser His Gln His Asp Gly Met Glu Ala Tyr Val Lys Val Asp Ser Cys
        335                 340                 345

cca gag gaa ccc caa cta cga atg aaa aat aat gaa gaa gcg gaa gac    1767
Pro Glu Glu Pro Gln Leu Arg Met Lys Asn Asn Glu Glu Ala Glu Asp
    350                 355                 360

tat gat gat gat ctt act gat tct gaa atg gat gtg gtc agg ttt gat    1815
Tyr Asp Asp Asp Leu Thr Asp Ser Glu Met Asp Val Val Arg Phe Asp
365                 370                 375                 380

gat gac aac tct cct tcc ttt atc caa att cgc tca gtt gcc aag aag    1863
Asp Asp Asn Ser Pro Ser Phe Ile Gln Ile Arg Ser Val Ala Lys Lys
                385                 390                 395

cat cct aaa act tgg gta cat tac att gct gct gaa gag gag gac tgg    1911
His Pro Lys Thr Trp Val His Tyr Ile Ala Ala Glu Glu Glu Asp Trp
            400                 405                 410

gac tat gct ccc tta gtc ctc gcc ccc gat gac aga agt tat aaa agt 1959
Asp Tyr Ala Pro Leu Val Leu Ala Pro Asp Asp Arg Ser Tyr Lys Ser
        415                 420                 425

caa tat ttg aac aat ggc cct cag cgg att ggt agg aag tac aaa aaa 2007
Gln Tyr Leu Asn Asn Gly Pro Gln Arg Ile Gly Arg Lys Tyr Lys Lys
    430                 435                 440

gtc cga ttt atg gca tac aca gat gaa acc ttt aag act cgt gaa gct 2055
Val Arg Phe Met Ala Tyr Thr Asp Glu Thr Phe Lys Thr Arg Glu Ala
445                 450                 455                 460

att cag cat gaa tca gga atc ttg gga cct tta ctt tat ggg gaa gtt 2103
Ile Gln His Glu Ser Gly Ile Leu Gly Pro Leu Leu Tyr Gly Glu Val
                465                 470                 475

gga gac aca ctg ttg att ata ttt aag aat caa gca agc aga cca tat 2151
Gly Asp Thr Leu Leu Ile Ile Phe Lys Asn Gln Ala Ser Arg Pro Tyr
            480                 485                 490

aac atc tac cct cac gga atc act gat gtc cgt cct ttg tat tca agg 2199
Asn Ile Tyr Pro His Gly Ile Thr Asp Val Arg Pro Leu Tyr Ser Arg
        495                 500                 505

aga tta cca aaa ggt gta aaa cat ttg aag gat ttt cca att ctg cca 2247
Arg Leu Pro Lys Gly Val Lys His Leu Lys Asp Phe Pro Ile Leu Pro
    510                 515                 520

gga gaa ata ttc aaa tat aaa tgg aca gtg act gta gaa gat ggg cca 2295
Gly Glu Ile Phe Lys Tyr Lys Trp Thr Val Thr Val Glu Asp Gly Pro
525                 530                 535                 540

act aaa tca gat cct cgg tgc ctg acc cgc tat tac tct agt ttc gtt 2343
Thr Lys Ser Asp Pro Arg Cys Leu Thr Arg Tyr Tyr Ser Ser Phe Val
                545                 550                 555

aat atg gag aga gat cta gct tca gga ctc att ggc cct ctc ctc atc 2391
Asn Met Glu Arg Asp Leu Ala Ser Gly Leu Ile Gly Pro Leu Leu Ile
            560                 565                 570

tgc tac aaa gaa tct gta gat caa aga gga aac cag ata atg tca gac 2439
Cys Tyr Lys Glu Ser Val Asp Gln Arg Gly Asn Gln Ile Met Ser Asp
        575                 580                 585

aag agg aat gtc atc ctg ttt tct gta ttt gat gag aac cga agc tgg 2487
Lys Arg Asn Val Ile Leu Phe Ser Val Phe Asp Glu Asn Arg Ser Trp
    590                 595                 600

tac ctc aca gag aat ata caa cgc ttt ctc ccc aat cca gct gga gtg 2535
Tyr Leu Thr Glu Asn Ile Gln Arg Phe Leu Pro Asn Pro Ala Gly Val
605                 610                 615                 620

cag ctt gag gat cca gag ttc caa gcc tcc aac atc atg cac agc atc 2583
Gln Leu Glu Asp Pro Glu Phe Gln Ala Ser Asn Ile Met His Ser Ile
                625                 630                 635

aat ggc tat gtt ttt gat agt ttg cag ttg tca gtt tgt ttg cat gag 2631
Asn Gly Tyr Val Phe Asp Ser Leu Gln Leu Ser Val Cys Leu His Glu
            640                 645                 650

gtg gca tac tgg tac att cta agc att gga gca cag act gac ttc ctt 2679
Val Ala Tyr Trp Tyr Ile Leu Ser Ile Gly Ala Gln Thr Asp Phe Leu
        655                 660                 665

tct gtc ttc ttc tct gga tat acc ttc aaa cac aaa atg gtc tat gaa 2727
Ser Val Phe Phe Ser Gly Tyr Thr Phe Lys His Lys Met Val Tyr Glu
    670                 675                 680

gac aca ctc acc cta ttc cca ttc tca gga gaa act gtc ttc atg tcg 2775
Asp Thr Leu Thr Leu Phe Pro Phe Ser Gly Glu Thr Val Phe Met Ser
685                 690                 695                 700

atg gaa aac cca ggt cta tgg att ctg ggg tgc cac aac tca gac ttt 2823
Met Glu Asn Pro Gly Leu Trp Ile Leu Gly Cys His Asn Ser Asp Phe
                705                 710                 715

cgg aac aga ggc atg acc gcc tta ctg aag gtt tct agt tgt gac aag 2871
Arg Asn Arg Gly Met Thr Ala Leu Leu Lys Val Ser Ser Cys Asp Lys
            720                 725                 730

aac act ggt gat tat tac gag gac agt tat gaa gat att tca gca tac 2919
Asn Thr Gly Asp Tyr Tyr Glu Asp Ser Tyr Glu Asp Ile Ser Ala Tyr
        735                 740                 745

ttg ctg agt aaa aac aat gcc att gaa cca aga agc ttc tcc cag aat 2967
Leu Leu Ser Lys Asn Asn Ala Ile Glu Pro Arg Ser Phe Ser Gln Asn
    750                 755                 760

tca aga cat caa gct tat cga tac cgt cga ggg gaa ata act cgt act 3015
Ser Arg His Gln Ala Tyr Arg Tyr Arg Arg Gly Glu Ile Thr Arg Thr
765                 770                 775                 780

act ctt cag tca gat caa gag gaa att gac tat gat gat acc ata tca 3063
Thr Leu Gln Ser Asp Gln Glu Glu Ile Asp Tyr Asp Asp Thr Ile Ser
                785                 790                 795

gtt gaa atg aag aag gaa gat ttt gac att tat gat gag gat gaa aat 3111
Val Glu Met Lys Lys Glu Asp Phe Asp Ile Tyr Asp Glu Asp Glu Asn
            800                 805                 810

cag agc ccc cgc agc ttt caa aag aaa aca cga cac tat ttt att gct 3159
Gln Ser Pro Arg Ser Phe Gln Lys Lys Thr Arg His Tyr Phe Ile Ala
        815                 820                 825

gca gtg gag agg ctc tgg gat tat ggg atg agt agc tcc cca cat gtt 3207
Ala Val Glu Arg Leu Trp Asp Tyr Gly Met Ser Ser Ser Pro His Val
    830                 835                 840

cta aga aac agg gct cag agt ggc agt gtc cct cag ttc aag aaa gtt 3255
Leu Arg Asn Arg Ala Gln Ser Gly Ser Val Pro Gln Phe Lys Lys Val
845                 850                 855                 860

gtt ttc cag gaa ttt act gat ggc tcc ttt act cag ccc tta tac cgt 3303
Val Phe Gln Glu Phe Thr Asp Gly Ser Phe Thr Gln Pro Leu Tyr Arg
                865                 870                 875

gga gaa cta aat gaa cat ttg gga ctc ctg ggg cca tat ata aga gca 3351
Gly Glu Leu Asn Glu His Leu Gly Leu Leu Gly Pro Tyr Ile Arg Ala
            880                 885                 890

gaa gtt gaa gat aat atc atg gta act ttc aga aat cag gcc tct cgt 3399
Glu Val Glu Asp Asn Ile Met Val Thr Phe Arg Asn Gln Ala Ser Arg
        895                 900                 905

ccc tat tcc ttc tat tct agc ctt att tct tat gag gaa gat cag agg 3447
Pro Tyr Ser Phe Tyr Ser Ser Leu Ile Ser Tyr Glu Glu Asp Gln Arg
    910                 915                 920

caa gga gca gaa cct aga aaa aac ttt gtc aag cct aat gaa acc aaa    3495
Gln Gly Ala Glu Pro Arg Lys Asn Phe Val Lys Pro Asn Glu Thr Lys
925                 930                 935                 940

act tac ttt tgg aaa gtg caa cat cat atg gca ccc act aaa gat gag    3543
Thr Tyr Phe Trp Lys Val Gln His His Met Ala Pro Thr Lys Asp Glu
                945                 950                 955

ttt gac tgc aaa gcc tgg gct tat ttc tct gat gtt gac ctg gaa aaa    3591
Phe Asp Cys Lys Ala Trp Ala Tyr Phe Ser Asp Val Asp Leu Glu Lys
            960                 965                 970

gat gtg cac tca ggc ctg att gga ccc ctt ctg gtc tgc cac act aac    3639
Asp Val His Ser Gly Leu Ile Gly Pro Leu Leu Val Cys His Thr Asn
        975                 980                 985

aca ctg aac cct gct cat ggg aga caa gtg aca gta cag gaa ttt gct    3687
Thr Leu Asn Pro Ala His Gly Arg Gln Val Thr Val Gln Glu Phe Ala
    990                 995                 1000

ctg ttt ttc acc atc ttt gat gag acc aaa agc tgg tac ttc act gaa    3735
Leu Phe Phe Thr Ile Phe Asp Glu Thr Lys Ser Trp Tyr Phe Thr Glu
1005                1010                1015                1020

aat atg gaa aga aac tgc agg gct ccc tgc aat atc cag atg gaa gat    3783
Asn Met Glu Arg Asn Cys Arg Ala Pro Cys Asn Ile Gln Met Glu Asp
                1025                1030                1035

ccc act ttt aaa gag aat tat cgc ttc cat gca atc aat ggc tac ata    3831
Pro Thr Phe Lys Glu Asn Tyr Arg Phe His Ala Ile Asn Gly Tyr Ile
            1040                1045                1050

atg gat aca cta cct ggc tta gta atg gct cag gat caa agg att cga    3879
Met Asp Thr Leu Pro Gly Leu Val Met Ala Gln Asp Gln Arg Ile Arg
        1055                1060                1065

tgg tat ctg ctc agc atg ggc agc aat gaa aac atc cat tct att cat    3927
Trp Tyr Leu Leu Ser Met Gly Ser Asn Glu Asn Ile His Ser Ile His
    1070                1075                1080

ttc agt gga cat gtg ttc act gta cga aaa aaa gag gag tat aaa atg    3975
Phe Ser Gly His Val Phe Thr Val Arg Lys Lys Glu Glu Tyr Lys Met
1085                1090                1095                1100

gca ctg tac aat ctc tat cca ggt gtt ttt gag aca gtg gaa atg tta    4023
Ala Leu Tyr Asn Leu Tyr Pro Gly Val Phe Glu Thr Val Glu Met Leu
                1105                1110                1115

cca tcc aaa gct gga att tgg cgg gtg gaa tgc ctt att ggc gag cat    4071
Pro Ser Lys Ala Gly Ile Trp Arg Val Glu Cys Leu Ile Gly Glu His
            1120                1125                1130

cta cat gct ggg atg aac aca ctt ttt ctg gtg tac agc aat aag tgt    4119
Leu His Ala Gly Met Asn Thr Leu Phe Leu Val Tyr Ser Asn Lys Cys
        1135                1140                1145

cag act ccc ctg gga atg gct tct gga cac att aga gat ttt cag att    4167
Gln Thr Pro Leu Gly Met Ala Ser Gly His Ile Arg Asp Phe Gln Ile
    1150                1155                1160

aca gct tca gga caa tat gga cag tgg gcc cca aag ctg gcc aga ctt    4215
Thr Ala Ser Gly Gln Tyr Gly Gln Trp Ala Pro Lys Leu Ala Arg Leu
1165                1170                1175                1180

cat tat tcc gga tca atc aat gcc tgg agc acc aag gag ccc ttt tct 4263
His Tyr Ser Gly Ser Ile Asn Ala Trp Ser Thr Lys Glu Pro Phe Ser
                1185                1190                1195

tgg atc aag gtg gat ctg ttg gca cca atg att att cac ggc atc aag 4311
Trp Ile Lys Val Asp Leu Leu Ala Pro Met Ile Ile His Gly Ile Lys
            1200                1205                1210

acc cag ggt gcc cgt cag aag ttc tcc agc ctc tac atc tct cag ttt 4359
Thr Gln Gly Ala Arg Gln Lys Phe Ser Ser Leu Tyr Ile Ser Gln Phe
        1215                1220                1225

atc atc atg tat agt ctt gat ggg aag aag tgg cag act tat cga gga 4407
Ile Ile Met Tyr Ser Leu Asp Gly Lys Lys Trp Gln Thr Tyr Arg Gly
    1230                1235                1240

aat tcc act gga acc tta atg gtc ttc ttt ggc aat gtg gat tca tct 4455
Asn Ser Thr Gly Thr Leu Met Val Phe Phe Gly Asn Val Asp Ser Ser
1245                1250                1255                1260

ggg ata aaa cac aat att ttt aac cct cca att att gct cga tac atc 4503
Gly Ile Lys His Asn Ile Phe Asn Pro Pro Ile Ile Ala Arg Tyr Ile
                1265                1270                1275

cgt ttg cac cca act cat tat agc att cgc agc act ctt cgc atg gag 4551
Arg Leu His Pro Thr His Tyr Ser Ile Arg Ser Thr Leu Arg Met Glu
            1280                1285                1290

ttg atg ggc tgt gat tta aat agt tgc agc atg cca ttg gga atg gag 4599
Leu Met Gly Cys Asp Leu Asn Ser Cys Ser Met Pro Leu Gly Met Glu
        1295                1300                1305

agt aaa gca ata tca gat gca cag att act gct tca tcc tac ttt acc 4647
Ser Lys Ala Ile Ser Asp Ala Gln Ile Thr Ala Ser Ser Tyr Phe Thr
    1310                1315                1320

aat atg ttt gcc acc tgg tct cct tca aaa gct cga ctt cac ctc caa 4695
Asn Met Phe Ala Thr Trp Ser Pro Ser Lys Ala Arg Leu His Leu Gln
1325                1330                1335                1340

ggg agg agt aat gcc tgg aga cct cag gag aat aat cca aaa gag tgg 4743
Gly Arg Ser Asn Ala Trp Arg Pro Gln Glu Asn Asn Pro Lys Glu Trp
                1345                1350                1355

ctg caa gtg gac ttc cag aag aca atg aaa gtc aca gga gta act act 4791
Leu Gln Val Asp Phe Gln Lys Thr Met Lys Val Thr Gly Val Thr Thr
            1360                1365                1370

cag gga gta aaa tct ctg ctt acc agc atg tat gtg aag gag ttc ctc 4839
Gln Gly Val Lys Ser Leu Leu Thr Ser Met Tyr Val Lys Glu Phe Leu
        1375                1380                1385

atc tcc agc agt caa gat ggc cat cag tgg acc ctc ttt ttt cag aat 4887
Ile Ser Ser Ser Gln Asp Gly His Gln Trp Thr Leu Phe Phe Gln Asn
    1390                1395                1400

ggc aaa gta aag gtt ttt cag gga aat caa gac tcc ttc aca cct gtg 4935
Gly Lys Val Lys Val Phe Gln Gly Asn Gln Asp Ser Phe Thr Pro Val
1405                1410                1415                1420

gtg aac tct cta gac cca ccg tta ctg act cgc tac ctt cga att cac 4983
Val Asn Ser Leu Asp Pro Pro Leu Leu Thr Arg Tyr Leu Arg Ile His
                1425                1430                1435

ccc cag agt tgg gtg cac cag att gcc ctg agg atg gag gtt ctg ggc 5031
Pro Gln Ser Trp Val His Gln Ile Ala Leu Arg Met Glu Val Leu Gly
            1440                1445                1450

tgc gag gca cag gac ctc tac tgagcggccg cgactctact agaggatctt 5082
Cys Glu Ala Gln Asp Leu Tyr
        1455

tgtgaaggaa ccttacttct gtggtgtgac ataattggac aaactaccta cagagattta 5142

aagctctaag gtaaatataa aatttttaag tgtataatgt gttaaactac tgattctaat 5202

tgtttgtgta ttttagattc caacctatgg aactgatgaa tgggagcagt ggtggaatgc 5262

ctttaatgag gaaaacctgt tttgctcaga agaaatgcca tctagtgatg atgaggctac 5322

tgctgactct caacattcta ctcctccaaa aaagaagaga aaggtagaag accccaagga 5382

ctttccttca gaattgctaa gttttttgag tcatgctgtg tttagtaata gaactcttgc 5442

ttgctttgct atttacacca caaaggaaaa agctgcactg ctatacaaga aaattatgga 5502

aaaatattct gtaaccttta taagtaggca taacagttat aatcataaca tactgttttt 5562

tcttactcca cacaggcata gagtgtctgc tattaataac tatgctcaaa aattgtgtac 5622

ctttagcttt ttaatttgta aaggggttaa taaggaatat ttgatgtata gtgccttgac 5682

tagagatcat aatcagccat accacatttg tagaggtttt acttgcttta aaaaacctcc 5742

cacacctccc cctgaacctg aaacataaaa tgaatgcaat tgttgttgtt aacttgttta 5802

ttgcagctta taatggttac aaataaagca atagcatcac aaatttcaca aataaagcat 5862

ttttttcact gcattctagt tgtggtttgt ccaaactcat caatgtatct tatcatgtct 5922

ggatccccgg gtaccctcta gagcgaatta attcactggc cgtcgtttta caacgtcgtg 5982

actgggaaaa ccctggcgtt acccaactta atcgccttgc agcacatccc cctttcgcca 6042

gctggcgtaa tagcgaagag gcccgcaccg atcgcccttc ccaacagttg cgcagcctga 6102

atggcgaatg gcgcctgatg cggtattttc tccttacgca tctgtgcggt atttcacacc 6162

gcatatggtg cactctcagt acaatctgct ctgatgccgc atagttaagc cagccccgac 6222

acccgccaac acccgctgac gcgccctgac gggcttgtct gctcccggca tccgcttaca 6282

gacaagctgt gaccgtctcc gggagctgca tgtgtcagag gttttcaccg tcatcaccga 6342

aacgcgcgag acgaaagggg gggtaccagc ttcgtagcta gaacatcatg ttctgggata 6402

tcagcttcgt agctagaaca tcatgttctg gtacccccct cgtgatacgc ctatttttat 6462

aggttaatgt catgataata atggtttctt agacgtcagg tggcactttt cggggaaatg 6522

tgcgcggaac ccctatttgt ttatttttct aaatacattc aaatatgtat ccgctcatga 6582

gacaataacc ctgataaatg cttcaataat attgaaaaag gaagagtatg agtattcaac 6642

atttccgtgt cgcccttatt cccttttttg cggcattttg ccttcctgtt tttgctcacc 6702

cagaaacgct ggtgaaagta aaagatgctg aagatcagtt gggtgcacga gtgggttaca 6762

tcgaactgga tctcaacagc ggtaagatcc ttgagagttt tcgccccgaa gaacgttttc 6822

caatgatgag cacttttaaa gttctgctat gtggcgcggt attatcccgt attgacgccg 6882
ggcaagagca actcggtcgc cgcatacact attctcagaa tgacttggtt gagtactcac 6942
cagtcacaga aaagcatctt acggatggca tgacagtaag agaattatgc agtgctgcca 7002
taaccatgag tgataacact gcggccaact tacttctgac aacgatcgga ggaccgaagg 7062
agctaaccgc ttttttgcac aacatggggg atcatgtaac tcgccttgat cgttgggaac 7122
cggagctgaa tgaagccata ccaaacgacg agcgtgacac cacgatgcct gtagcaatgg 7182
caacaacgtt gcgcaaacta ttaactggcg aactacttac tctagcttcc cggcaacaat 7242
taatagactg gatggaggcg gataaagttg caggaccact tctgcgctcg gcccttccgg 7302
ctggctggtt tattgctgat aaatctggag ccggtgagcg tgggtctcgc ggtatcattg 7362
cagcactggg gccagatggt aagccctccc gtatcgtagt tatctacacg acggggagtc 7422
aggcaactat ggatgaacga aatagacaga tcgctgagat aggtgcctca ctgattaagc 7482
attggtaact gtcagaccaa gtttactcat atatacttta gattgattta aaacttcatt 7542
tttaatttaa aaggatctag gtgaagatcc tttttgataa tctcatgacc aaaatccctt 7602
aacgtgagtt ttcgttccac tgagcgtcag accccgtaga aaagatcaaa ggatcttctt 7662
gagatccttt ttttctgcgc gtaatctgct gcttgcaaac aaaaaaacca ccgctaccag 7722
cggtggtttg tttgccggat caagagctac caactctttt tccgaaggta actggcttca 7782
gcagagcgca gataccaaat actgtcttct agtgtagccg tagttaggcc accacttcaa 7842
gaactctgta gcaccgccta catacctcgc tctgctaatc ctgttaccag tggctgctgc 7902
cagtggcgat aagtcgtgtc ttaccgggtt ggactcaaga cgatagttac cggataaggc 7962
gcagcggtcg ggctgaacgg ggggttcgtg cacacagccc agcttggagc gaacgaccta 8022
caccgaactg agatacctac agcgtgagct atgagaaagc gccacgcttc ccgaagggag 8082
aaaggcggac aggtatccgg taagcggcag ggtcggaaca ggagagcgca cgagggagct 8142
tccaggggga aacgcctggt atctttatag tcctgtcggg tttcgccacc tctgacttga 8202
gcgtcgattt ttgtgatgct cgtcaggggg gcggagccta tggaaaaacg ccagcaacgc 8262
ggccttttta cggttcctgg ccttttgctg gccttttgct cacatgttct ttcctgcgtt 8322
atcccctgat tctgtggata accgtattac cgcctttgag tgagctgata ccgctcgccg 8382
cagccgaacg accgagcgca gcgagtcagt gagcgaggaa gcggaagagc gcccaatacg 8442
caaaccgcct ctccccgcgc gttggccgat tcattaatgc agctggcacg acaggtttcc 8502
cgactggaaa gcgggcagtg agcgcaacgc aattaatgtg agttagctca ctcattaggc 8562
accccaggct ttacacttta tgcttccggc tcgtatgttg tgtggaattg tgagcggata 8622
acaatttcac acaggaaaca gctatgacca tgattacgcc aagctctcta gagctctaga 8682
gctctagagc tctagagagc ttgcatgcct gcaggtcg 8720

<210> 5
<211> 1459
<212> PRT
<213> Artificial Sequence
<223> Description of Artificial Sequence: pTGF8-1

<400> 5
Met Gln Ile Glu Leu Ser Thr Cys Phe Phe Leu Cys Leu Leu Arg Phe
1               5                   10                  15

Cys Phe Ser Ala Thr Arg Arg Tyr Tyr Leu Gly Ala Val Glu Leu Ser
            20                  25                  30

Trp Asp Tyr Met Gln Ser Asp Leu Gly Glu Leu Pro Val Asp Ala Arg
        35                  40                  45

Phe Pro Pro Arg Val Pro Lys Ser Phe Pro Phe Asn Thr Ser Val Val
    50                  55                  60

Tyr Lys Lys Thr Leu Phe Val Glu Phe Thr Asp His Leu Phe Asn Ile
65                  70                  75                  80

Ala Lys Pro Arg Pro Pro Trp Met Gly Leu Leu Gly Pro Thr Ile Gln
                85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser
                165                 170                 175

Tyr Leu Ser His Ala Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp
225                 230                 235                 240

Ala Ala Ser Ala Arg Ala Trp Pro Lys Met His Thr Val Asn Gly Tyr
                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300

Leu Glu Ile Ser Pro Ile Thr Phe Leu Thr Ala Gln Thr Leu Leu Met
305                 310                 315                 320

Asp Leu Gly Gln Phe Leu Leu Phe Cys His Ile Ser Ser His Gln His
                325                 330                 335

Asp Gly Met Glu Ala Tyr Val Lys Val Asp Ser Cys Pro Glu Glu Pro
            340                 345                 350

Gln Leu Arg Met Lys Asn Asn Glu Glu Ala Glu Asp Tyr Asp Asp Asp
        355                 360                 365

Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400

Trp Val His Tyr Ile Ala Ala Glu Glu Glu Asp Trp Asp Tyr Ala Pro
                405                 410                 415

Leu Val Leu Ala Pro Asp Asp Arg Ser Tyr Lys Ser Gln Tyr Leu Asn
            420                 425                 430

Asn Gly Pro Gln Arg Ile Gly Arg Lys Tyr Lys Lys Val Arg Phe Met
        435                 440                 445

Ala Tyr Thr Asp Glu Thr Phe Lys Thr Arg Glu Ala Ile Gln His Glu
    450                 455                 460

Ser Gly Ile Leu Gly Pro Leu Leu Tyr Gly Glu Val Gly Asp Thr Leu
465                 470                 475                 480

Leu Ile Ile Phe Lys Asn Gln Ala Ser Arg Pro Tyr Asn Ile Tyr Pro
                485                 490                 495

His Gly Ile Thr Asp Val Arg Pro Leu Tyr Ser Arg Arg Leu Pro Lys
            500                 505                 510

Gly Val Lys His Leu Lys Asp Phe Pro Ile Leu Pro Gly Glu Ile Phe
        515                 520                 525

Lys Tyr Lys Trp Thr Val Thr Val Glu Asp Gly Pro Thr Lys Ser Asp
    530                 535                 540

Pro Arg Cys Leu Thr Arg Tyr Tyr Ser Ser Phe Val Asn Met Glu Arg
545                 550                 555                 560

Asp Leu Ala Ser Gly Leu Ile Gly Pro Leu Leu Ile Cys Tyr Lys Glu
                565                 570                 575

Ser Val Asp Gln Arg Gly Asn Gln Ile Met Ser Asp Lys Arg Asn Val
            580                 585                 590

Ile Leu Phe Ser Val Phe Asp Glu Asn Arg Ser Trp Tyr Leu Thr Glu
        595                 600                 605

Asn Ile Gln Arg Phe Leu Pro Asn Pro Ala Gly Val Gln Leu Glu Asp
    610                 615                 620

Pro Glu Phe Gln Ala Ser Asn Ile Met His Ser Ile Asn Gly Tyr Val
625                 630                 635                 640

Phe Asp Ser Leu Gln Leu Ser Val Cys Leu His Glu Val Ala Tyr Trp
                645                 650                 655

Tyr Ile Leu Ser Ile Gly Ala Gln Thr Asp Phe Leu Ser Val Phe Phe
            660                 665                 670

Ser Gly Tyr Thr Phe Lys His Lys Met Val Tyr Glu Asp Thr Leu Thr
        675                 680                 685

Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720

Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Ser Arg His Gln
        755                 760                 765

Ala Tyr Arg Tyr Arg Arg Gly Glu Ile Thr Arg Thr Thr Leu Gln Ser
    770                 775                 780

Asp Gln Glu Glu Ile Asp Tyr Asp Asp Thr Ile Ser Val Glu Met Lys
785                 790                 795                 800

Lys Glu Asp Phe Asp Ile Tyr Asp Glu Asp Glu Asn Gln Ser Pro Arg
                805                 810                 815

Ser Phe Gln Lys Lys Thr Arg His Tyr Phe Ile Ala Ala Val Glu Arg
            820                 825                 830

Leu Trp Asp Tyr Gly Met Ser Ser Ser Pro His Val Leu Arg Asn Arg
        835                 840                 845

Ala Gln Ser Gly Ser Val Pro Gln Phe Lys Lys Val Val Phe Gln Glu
    850                 855                 860

Phe Thr Asp Gly Ser Phe Thr Gln Pro Leu Tyr Arg Gly Glu Leu Asn
865                 870                 875                 880

Glu His Leu Gly Leu Leu Gly Pro Tyr Ile Arg Ala Glu Val Glu Asp
                885                 890                 895

Asn Ile Met Val Thr Phe Arg Asn Gln Ala Ser Arg Pro Tyr Ser Phe
            900                 905                 910

Tyr Ser Ser Leu Ile Ser Tyr Glu Glu Asp Gln Arg Gln Gly Ala Glu
        915                 920                 925

Pro Arg Lys Asn Phe Val Lys Pro Asn Glu Thr Lys Thr Tyr Phe Trp
    930                 935                 940

Lys Val Gln His His Met Ala Pro Thr Lys Asp Glu Phe Asp Cys Lys
945                 950                 955                 960

Ala Trp Ala Tyr Phe Ser Asp Val Asp Leu Glu Lys Asp Val His Ser
                965                 970                 975

Gly Leu Ile Gly Pro Leu Leu Val Cys His Thr Asn Thr Leu Asn Pro
            980                 985                 990

Ala His Gly Arg Gln Val Thr Val Gln Glu Phe Ala Leu Phe Phe Thr
        995                 1000                1005

Ile Phe Asp Glu Thr Lys Ser Trp Tyr Phe Thr Glu Asn Met Glu Arg
    1010                1015                1020

Asn Cys Arg Ala Pro Cys Asn Ile Gln Met Glu Asp Pro Thr Phe Lys
025 1030 1035 1040

Glu Asn Tyr Arg Phe His Ala Ile Asn Gly Tyr Ile Met Asp Thr Leu
                1045                1050                1055

Pro Gly Leu Val Met Ala Gln Asp Gln Arg Ile Arg Trp Tyr Leu Leu
            1060                1065                1070

Ser Met Gly Ser Asn Glu Asn Ile His Ser Ile His Phe Ser Gly His
        1075                1080                1085

Val Phe Thr Val Arg Lys Lys Glu Glu Tyr Lys Met Ala Leu Tyr Asn
    1090                1095                1100

Leu Tyr Pro Gly Val Phe Glu Thr Val Glu Met Leu Pro Ser Lys Ala
105 1110 1115 1120

Gly Ile Trp Arg Val Glu Cys Leu Ile Gly Glu His Leu His Ala Gly
                1125                1130                1135

Met Asn Thr Leu Phe Leu Val Tyr Ser Asn Lys Cys Gln Thr Pro Leu
            1140                1145                1150

Gly Met Ala Ser Gly His Ile Arg Asp Phe Gln Ile Thr Ala Ser Gly
        1155                1160                1165

Gln Tyr Gly Gln Trp Ala Pro Lys Leu Ala Arg Leu His Tyr Ser Gly
    1170                1175                1180

Ser Ile Asn Ala Trp Ser Thr Lys Glu Pro Phe Ser Trp Ile Lys Val
185 1190 1195 1200

Asp Leu Leu Ala Pro Met Ile Ile His Gly Ile Lys Thr Gln Gly Ala
                1205                1210                1215

Arg Gln Lys Phe Ser Ser Leu Tyr Ile Ser Gln Phe Ile Ile Met Tyr
            1220                1225                1230

Ser Leu Asp Gly Lys Lys Trp Gln Thr Tyr Arg Gly Asn Ser Thr Gly
        1235                1240                1245

Thr Leu Met Val Phe Phe Gly Asn Val Asp Ser Ser Gly Ile Lys His
    1250                1255                1260

Asn Ile Phe Asn Pro Pro Ile Ile Ala Arg Tyr Ile Arg Leu His Pro
265 1270 1275 1280

Thr His Tyr Ser Ile Arg Ser Thr Leu Arg Met Glu Leu Met Gly Cys
                1285                1290                1295

Asp Leu Asn Ser Cys Ser Met Pro Leu Gly Met Glu Ser Lys Ala Ile
            1300                1305                1310

Ser Asp Ala Gln Ile Thr Ala Ser Ser Tyr Phe Thr Asn Met Phe Ala
        1315                1320                1325

Thr Trp Ser Pro Ser Lys Ala Arg Leu His Leu Gln Gly Arg Ser Asn
    1330                1335                1340

Ala Trp Arg Pro Gln Glu Asn Asn Pro Lys Glu Trp Leu Gln Val Asp
345 1350 1355 1360

Phe Gln Lys Thr Met Lys Val Thr Gly Val Thr Thr Gln Gly Val Lys
                1365                1370                1375

Ser Leu Leu Thr Ser Met Tyr Val Lys Glu Phe Leu Ile Ser Ser Ser
            1380                1385                1390

Gln Asp Gly His Gln Trp Thr Leu Phe Phe Gln Asn Gly Lys Val Lys
        1395                1400                1405

Val Phe Gln Gly Asn Gln Asp Ser Phe Thr Pro Val Val Asn Ser Leu
    1410                1415                1420

Asp Pro Pro Leu Leu Thr Arg Tyr Leu Arg Ile His Pro Gln Ser Trp
425                 1430                1435                1440

Val His Gln Ile Ala Leu Arg Met Glu Val Leu Gly Cys Glu Ala Gln
                1445                1450                1455

Asp Leu Tyr

<210> 6
<211> 8
<212> PRT
<213> Artificial Sequence

<220>
<223> Description of Artificial Sequence: B-domain
      linker peptide

<400> 6
Ser Phe Ser Gln Asn Ser Arg His
  1               5

<210> 7
<211> 8
<212> PRT
<213> Artificial Sequence

<220>
<223> Description of Artificial Sequence: B-domain
      linker peptide

<400> 7
Gln Ala Tyr Arg Tyr Arg Arg Gly
  1               5

<210> 8
<211> 16
<212> PRT
<213> Artificial Sequence

<220>
<223> Description of Artificial Sequence: B-domain
      linker peptide

<400> 8
Ser Phe Ser Gln Asn Ser Arg His Gln Ala Tyr Arg Tyr Arg Arg Gly
  1               5                  10                  15